# EUROPÄISCHE PATENTSCHRIFT

(11) **EP 0 779 884 B1**
(45) Veröffentlichungstag und Bekanntmachung des Hinweises auf die Patenterteilung: **03.05.2000**
(21) Anmeldenummer: 95931216.6
(22) Anmeldetag: 28.08.1995
(51) Int. Cl.: C07C 257/06, C07C 259/06, C07C 257/14, C07C 257/20, C07D 249/08, C07D 333/24, A01N 37/52, A01N 43/653, A01N 43/40, A01N 43/10

(54) **IMIDSÄUREDERIVATE UND IHRE VERWENDUNG ALS SCHÄDLINGSBEKÄMPFUNGSMITTEL**
IMIDIC ACID DERIVATIVES AND THEIR USE AS PESTICIDES
DERIVES D'IMIDO-ACIDES ET LEUR UTILISATION COMME PESTICIDES

(30) Priorität: 09.09.1994 DE 4432088; 10.03.1995 DE 19508573
(43) Veröffentlichungstag der Anmeldung: 25.06.1997
(73) Patentinhaber: BAYER AG, 51368 Leverkusen (DE)
(72) Erfinder: KUHNT, Dietmar, D-51399 Burscheid (DE); HEINEMANN, Ulrich, D-42799 Leichlingen (DE); GAYER, Herbert, D-40789 Monheim (DE); GERDES, Peter, D-52080 Aachen (DE); STETTER, Jörg, D-42115 Wuppertal (DE); TIEMANN, Ralf, D-51375 Leverkusen (DE); STENZEL, Klaus, D-40595 Düsseldorf (DE); DUTZMANN, Stefan, D-40721 Hilden (DE)
(86) Internationale Anmeldenummer: EP9503391
(87) Internationale Veröffentlichungsnummer: WO9607636

(56) Entgegenhaltungen:
- EP-A- 0 463 488
- EP-A- 0 528 681
- EP-A- 0 564 928
- EP-A- 0 644 183
- WO-A-94/22844
- WO-A-94/26700

## Beschreibung

Die vorliegende Erfindung betrifft neue Imidsäurederivate, mehrere Verfahren zu ihrer Herstellung und ihre Verwendung als Schädlingsbekämpfungsmittel.

Es ist bekannt, daß verschiedene substituierte Alkoximino- und Alkoxymethylenacetamide fungizide Eigenschaften besitzen (vgl. z.B. EP-A 398 692, EP-A 468 775, DE-A 40 30 038 WO-A 94/22844, WO-A 94/26700, EP-A-644 183 und WO-A 92/13 830).

Die Wirksamkeit dieser vorbekannten Verbindungen ist jedoch insbesondere bei niedrigen Aufwandmengen und Konzentrationen nicht in allen Anwendungsgebieten völlig zufriedenstellend.

Es wurden neue Imidsäurederivate der allgemeinen Formel (I) gefunden, 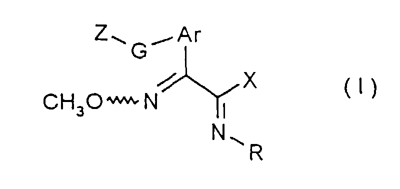 in welcher
- R: für Cyano steht;
- X: für Alkoxy, Alkylthio, Amino, Alkylamino oder Dialkylamino steht, oder
X und R gemeinsam mit dem Kohlenstoff- bzw. Stickstoff-Atom, an das sie gebunden sind, für einen Triazolrest der Formel 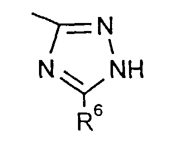 stehen, wobei
R⁶ für Alkyl, Hydroxy, Mercapto, Amino, Alkylamino oder Dialkylamino steht;
- Ar: für jeweils gegebenenfalls substituiertes Arylen oder Heteroarylen steht;
- G: für eine Einfachbindung für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-O-,
wobei
Q für Sauerstoff oder Schwefel steht,
n für die Zahlen 0, 1 oder 2 steht,
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht; und
- Z: für jeweils gegebenenfalls substituiertes Alkyl, Alkenyl, Alkinyl, Cycloalkyl, Aryl oder Heterocyclyl steht;
wobei die Verbindungen ausgenommen sind, in denen gleichzeitig R für Wasserstoff steht, G für Sauerstoff oder die Gruppierung -OCH₂- steht und Z für jeweils gegebenenfalls substituiertes Aryl oder 2-Pyridyl steht.

Weiterhin wurde gefunden, daß man die neuen Imidsäurederivate der allgemeinen Formel (I) erhält, wenn man
a) Nitrile der allgemeinen Formel (II) 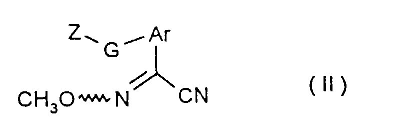 in welcher
   - Ar, G und Z: die oben angegebene Bedeutung haben,
   mit (Thio) Alkoholen der allgemeinen Formel (III)

   H-X¹ (III)

   in welcher
   - X¹: für Alkoxy oder Alkylthio steht,
   bzw. deren Alkalimetallsalzen, gegebenenfalls in Gegenwart eines Verdünnungsmittels und gegebenenfalls in Gegenwart eines Reaktionshilfsmittels umsetzt;
   oder
b) die gemäß Verfahren (a) erhältlichen Imidoester der allgemeinen Formel (Ia) 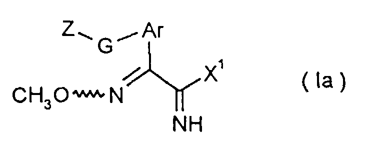 in welcher
   - Ar, G, Z und X¹: die oben angegebene Bedeutung haben,
   mit reaktiven Säurederivaten, insbesondere mit Säurehalogeniden der allgemeinen Formel (IV)

   Hal-R (IV)

   in welcher
   - R: die oben angegebene Bedeutung hat und
   - Hal: für Halogen steht,
   bzw. den entsprechenden Anhydriden, gegebenenfalls in Gegenwart eines Verdünnungsmittels und gegebenenfalls in Gegenwart eines Reaktionshilfsmittels umsetzt;
   oder
c) die gemäß Verfahren (a) erhältlichen Imidoester der allgemeinen Formel (Ia) 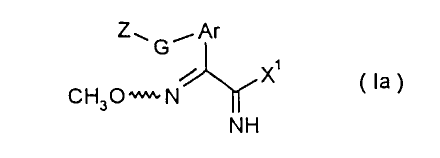 in welcher
   - Ar, G, Z und X¹: die oben angegebene Bedeutung haben,
   mit Cyanamid der Formel (V)

   NH₂-CN (V)

   gegebenenfalls in Gegenwart eines Verdünnungsmittels und gegebenenfalls in Gegenwart eines Reaktionshilfsmittels umsetzt;
   oder
d) die gemäß den Verfahren (b) und (c) erhältlichen Imidsäurederivate der allgemeinen Formel (Ib) 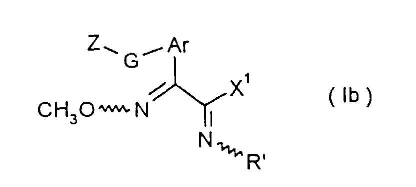 in welcher
   - Ar, G, Z und X¹: die oben angegebene Bedeutung haben und
   - R': für Cyano steht,
   mit Aminen der allgemeinen Formel (VI)

   H-X² (VI)

   in welcher
   - X²: für Amino, Alkylamino oder Dialkylamino steht,
   gegebenenfalls in Gegenwart eines Verdünnungsmittels umsetzt;
   oder
e) die gemäß den Verfahren (b) und (c) erhältlichen Imidsäurederivate der allgemeinen Formel (Ic) 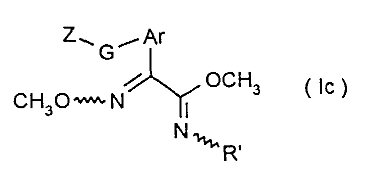 in welcher
   - Ar, G, Z und R': die oben angegebene Bedeutung haben,
   mit Hydrazin bzw. Hydrazinhydrat, gegebenenfalls in Gegenwart eines Verdünnungsmittels umsetzt;
   oder
f) die gemäß Verfahren (a) erhältlichen Imidoester der allgemeinen Formel (Ia) 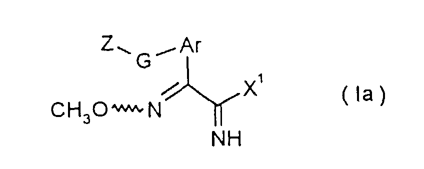 in welcher
   - Ar, G, Z und X¹: die oben angegebene Bedeutung haben,
   mit Aminen der allgemeinen Formel (VI)

   H-X² (VI)

   in welcher
   - X²: die oben angegebene Bedeutung hat,
   gegebenenfalls in Gegenwart eines Verdünnungsmittels umsetzt.
Schließlich wurde gefunden, daß die neuen Imidsäurederivate der allgemeinen Formel (I) starke fungizide Wirksamkeit zeigen.

Die erfindungsgemäßen Verbindungen können gegebenenfalls als Mischungen verschiedener möglicher isomerer Formen, insbesondere von E- und Z-Isomeren, vorliegen Es werden sowohl die E- als auch die Z-Isomeren wie auch beliebige Mischungen dieser Isomeren beansprucht.

Gegenstand der Erfindung sind vorzugsweise Verbindungen der Formel (I), in welcher
- R: für Cyano steht;
- X: für jeweils geradkettiges oder verzweigtes Alkoxy oder Alkylthio mit jeweils 1 bis 4 Kohlenstoffatomen;
Amino oder jeweils geradkettiges oder verzweigtes Alkylamino und Dialkylamino mit jeweils 1 bis 4 Kohlenstoffatomen in den einzelnen Alkylteilen steht.
X und R gemeinsam mit dem Kohlenstoff- bzw. Stickstoff-Atom, an das sie gebunden sind, für einen Triazolrest der Formel 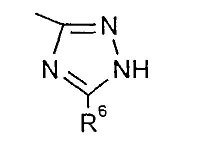 stehen, wobei
R⁶ für geradkettiges oder verzweigtes Alkyl mit 1 bis 4 Kohlenstoffatomen, Hydroxy, Mercapto, Amino oder jeweils geradkettiges oder verzweigtes Alkylamino und Dialkylamino mit jeweils 1 bis 4 Kohlenstoffatomen in den einzelnen Alkylteilen steht.
- Ar: für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen.
- G: für eine Einfachbindung, für Sauerstoff, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-,
wobei
n für die Zahlen 0, 1 oder 2 steht,
Q für Sauerstoff oder Schwefel steht,
R⁴ für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxy-carbonyl substituiertes Cycloalkyl mit 3 bis 6 Kohlenstoffatomen steht.
- Z: für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten aus der nachstehenden Aufzählung ausgewählt sind:
Sauerstoff (als Ersatz für zwei geminale Wasserstoffatome), Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano, Nitro, Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy;
wobei die oben per disclaimer ausgeschlossenen Verbindungen ausgenommen sind.

In den Definitionen sind die gesättigten oder ungesättigten Kohlenwasserstoffketten, wie Alkyl, Alkandiyl, Alkenyl oder Alkinyl, auch in Verknüpfung mit Heteroatomen, wie in Alkoxy, Alkylthio oder Alkylamino, jeweils geradkettig oder verzweigt.

Halogen steht im allgemeinen für Fluor, Chlor, Brom oder Iod, vorzugsweise für Fluor, Chlor oder Brom, insbesondere für Fluor oder Chlor.

Die Erfindung betrifft insbesondere Verbindungen der Formel (I), in welcher
- R: für Cyano steht;
- X: für Methoxy, Ethoxy, n- oder i-Propoxy, n-, i-, s- oder t-Butoxy; Methylthio, Ethylthio, n- oder i-Propylthio, n-, i-, s- oder t-Butylthio; Amino, Methylamino, Ethylamino, n- oder i-Propylamino; Dimethylamino, Methyl-ethyl-amino, Diethylamino, Methyl-n- oder i-Propylamino und Ethyl-n- oder i-Propylamino steht.
- X und R: gemeinsam mit dem Kohlenstoff- bzw. Stickstoff-Atom, an das sie gebunden sind, für einen Triazolrest der Formel 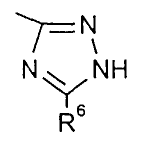 stehen, wobei
R⁶ für Methyl, Ethyl, n- oder i-Propyl, n-, i-, s- oder t-Butyl; Hydroxy; Mercapto; Amino, Methylamino, Ethylamino, n- oder i-Propylamino; Dimethylamino, Methyl-ethyl-amino, Diethylamino, Methyl-n- oder i-Propylamino und Ethyl-n- oder i-Propylamino steht.
- Ar: für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl.
- G: für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁵ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht.
- Z: für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethylamino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxy-carbonyl oder Ethoxycarbonyl substituiertes Phenyl, Phenoxy, Benzyl oder Benzyloxy;
wobei die oben per disclaimer ausgeschlossenen Verbindungen ausgenommen sind.

Eine besonders bevorzugte Gruppe erfindungsgemäßer Verbindungen sind diejenigen Verbindungen der Formel (I), in welcher
- R: für Cyano steht;
- X: für Methoxy, Ethoxy, Methylthio, Ethylthio, Amino, Methylamino, Ethylamino, Dimethylamino oder Diethylamino steht.
X und R gemeinsam mit dem Kohlenstoff- bzw. Stickstoff-Atom, an das sie gebunden sind, für einen Triazolrest der Formel 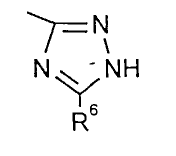 stehen, wobei
R⁶ für Methyl, Ethyl, Hydroxy, Mercapto oder Amino steht.
- Ar: für ortho-Phenylen, Pyridin-2,3-diyl oder Thiophen-2,3-diyl steht.
- G: für Sauerstoff, Methylen oder eine der nachstehenden Gruppierungen
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -O-C(R⁴)=N-O-CH₂-, -NH-C(R⁴)=N-O-CH₂-, -N(R⁵)- oder -CH₂-O-N=C(R⁴)- steht,
wobei
n für die Zahlen 0, 1 oder 2 steht,
R⁴ für Wasserstoff, Methyl oder Ethyl steht und
R⁵ für Wasserstoff, Methyl oder Ethyl steht.
- Z: für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy;
wobei die oben per disclaimer ausgeschlossenen Verbindungen ausgenommen sind.

Eine ganz besonders bevorzugte Gruppe von erfindungsgemäßen Verbindungen sind diejenigen Verbindungen der Formel (I), in welcher
- Ar: für ortho-Phenylen steht und
- R, X, G und Z: die oben genannten allgemeinen, vorzugsweise und besonders bevorzugten Bedeutungen haben.

Die oben aufgeführten allgemeinen oder in Vorzugsbereichen angegebenen Restedefinitionen gelten sowohl für die Endprodukte der Formel (I) als auch entsprechend für die jeweils zu Herstellung benötigten Ausgangsstoffe bzw. Zwischenprodukte.

Diese Restedefinitionen können untereinander, also auch zwischen den angegebenen Bereichen bevorzugter Verbindungen, beliebig kombiniert werden.

Beispiele für die erfindungsgemäßen Verbindungen sind in den Tabellen 1 bis 42 aufgeführt:

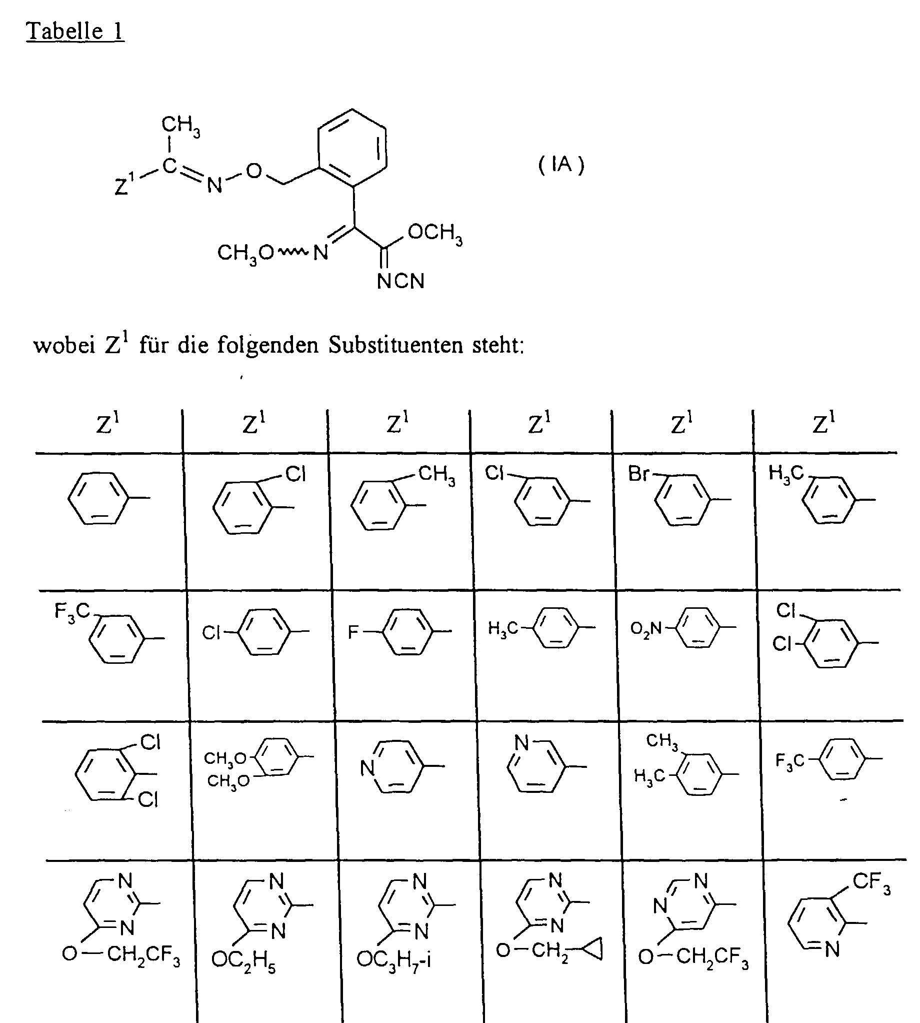

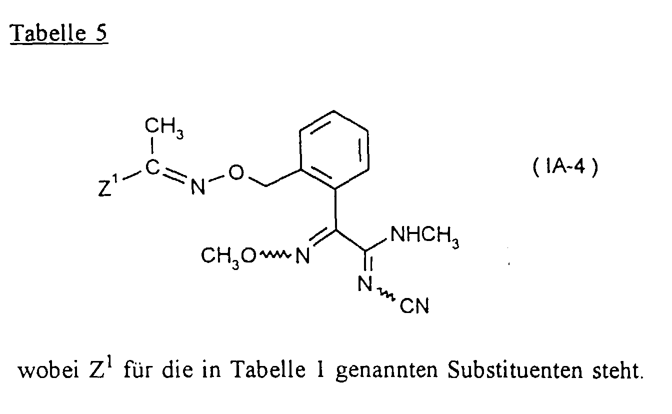

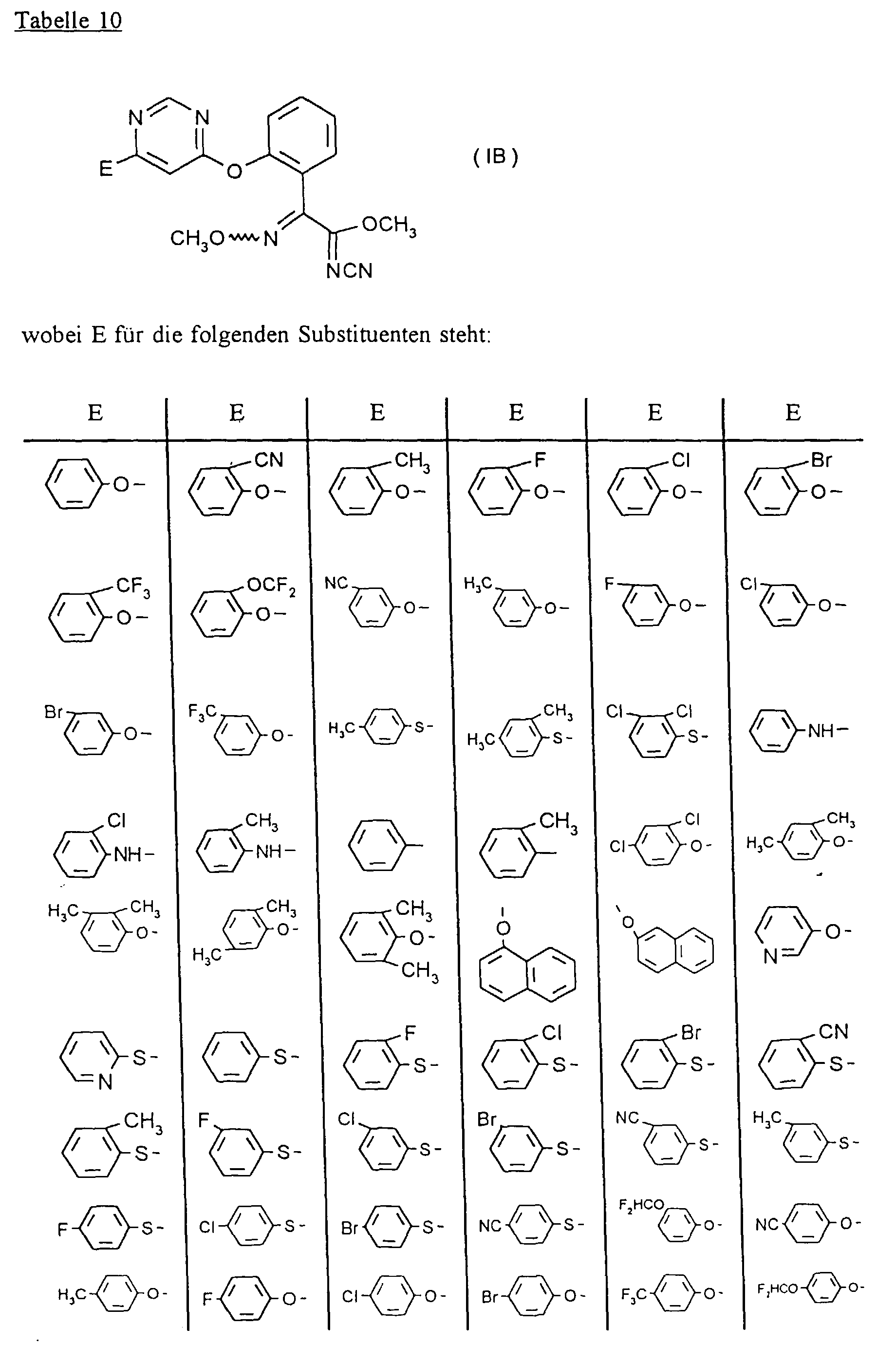

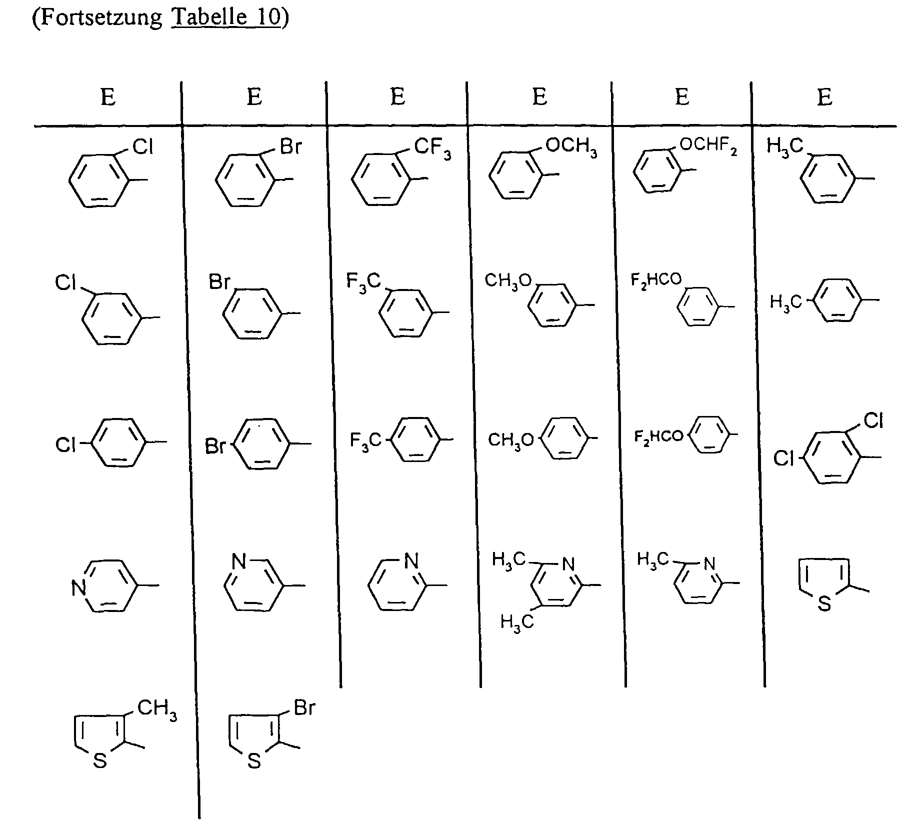

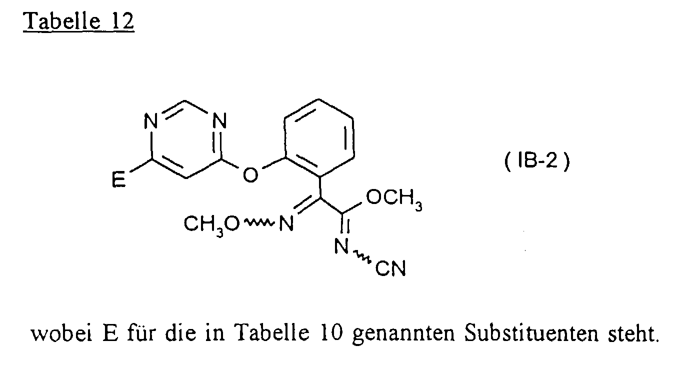

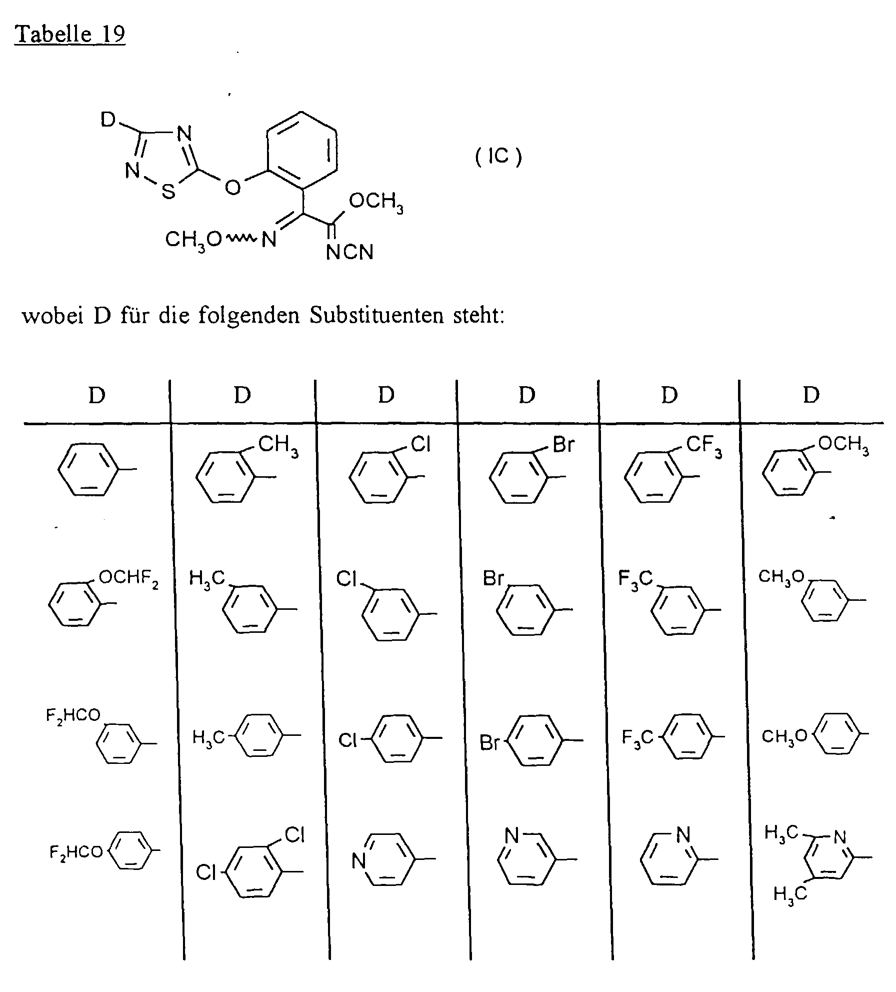

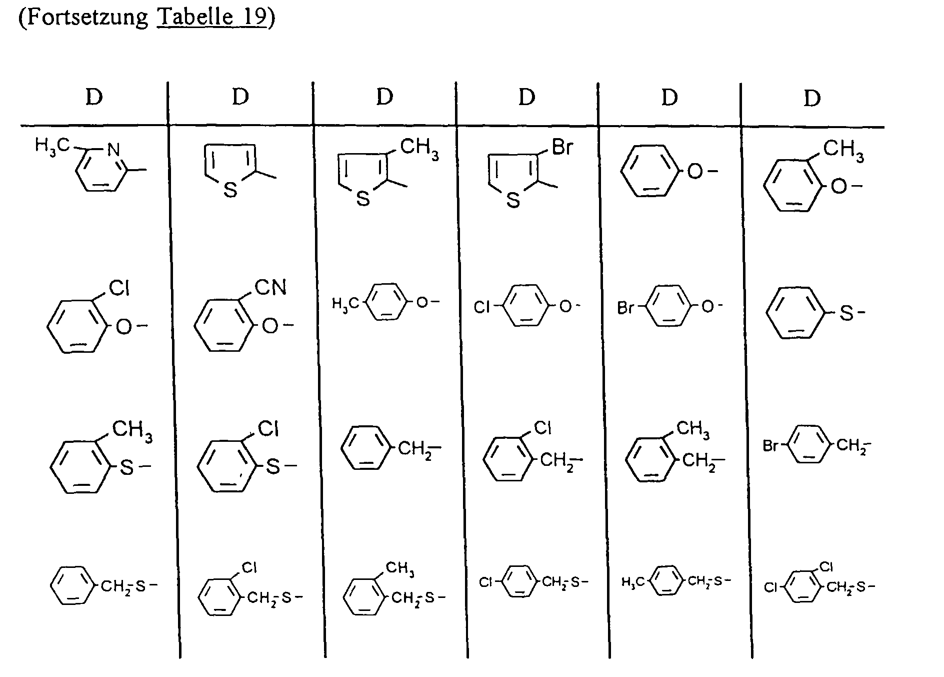

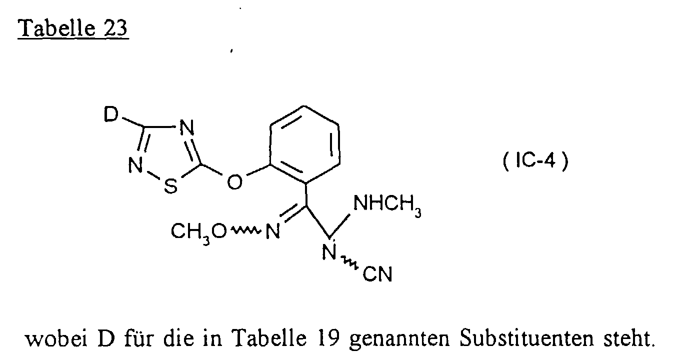

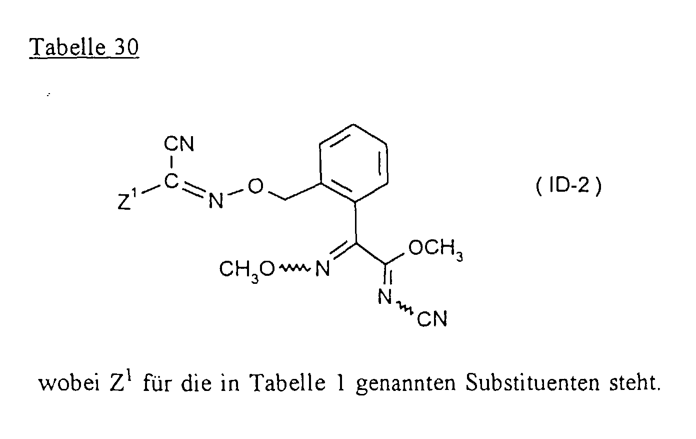

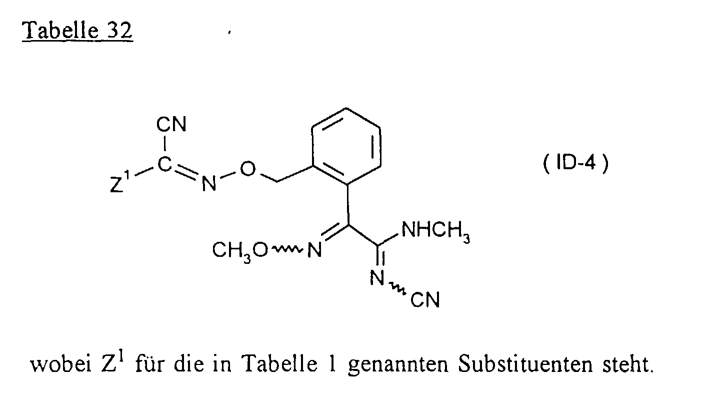

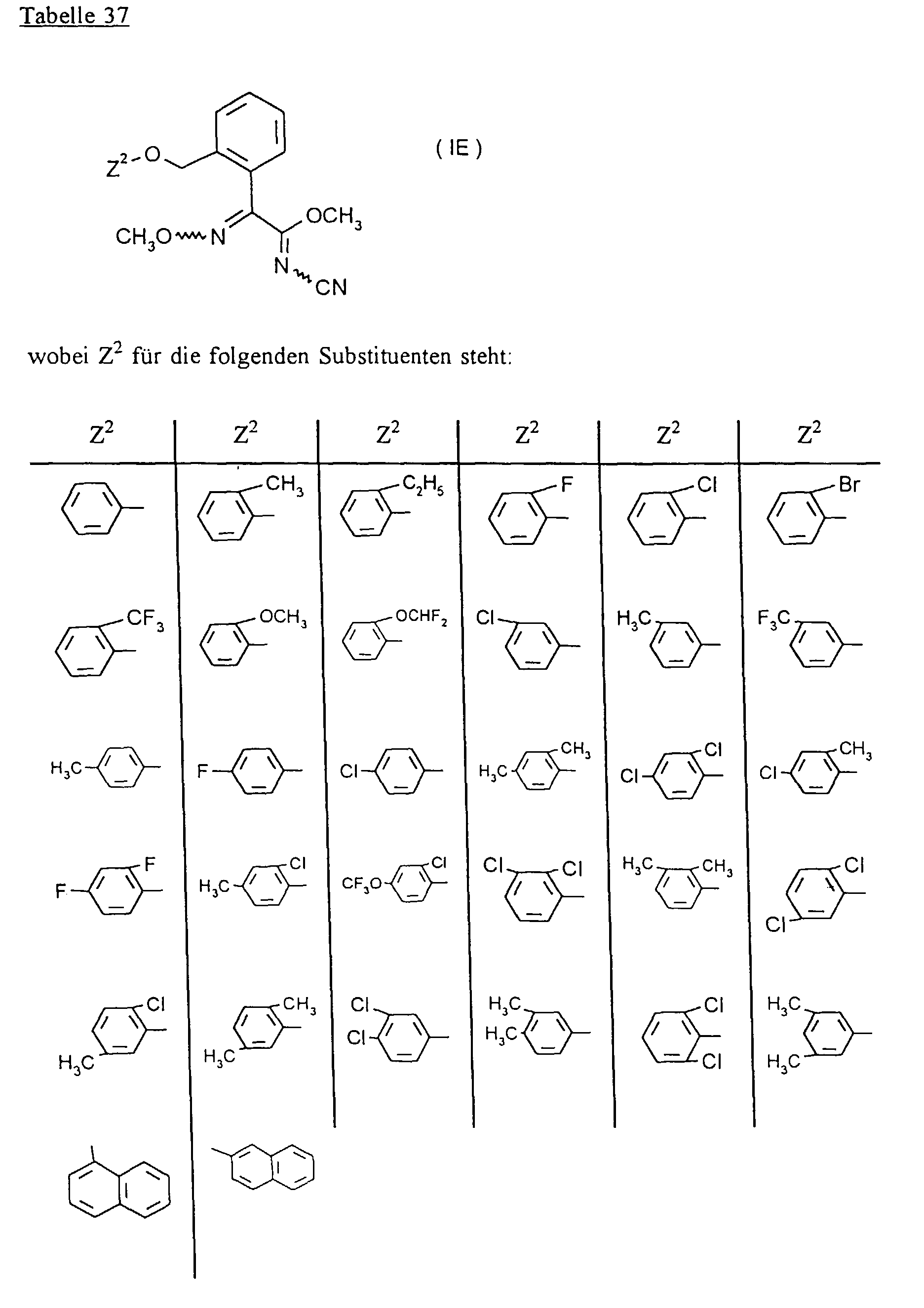

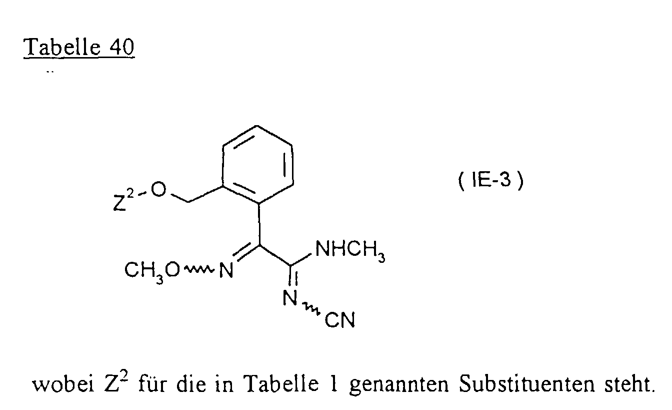

Die zur Durchführung des erfindungsgemäßen Verfahrens (a) als Ausgangsstoffe benötigten Nitrile sind duch die Formel (II) allgemein definiert. In der Formel (II) haben Ar, G und Z vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diese Substituenten genannt wurden.

Die Nitrile der Formel (II) sind noch nicht bekannt; sie können jedoch in allgemein bekannter Art und Weise erhalten werden, indem man Amide der allgemeinen Formel (VII) 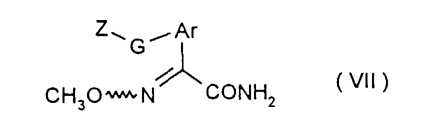 in welcher
- Ar, G und Z: die oben angegebene Bedeutung haben,
mit wasserabspaltenden Mitteln, wie beispielsweise Anhydriden, gegebenenfalls in Gegenwart eines Reaktionshilfsmittels, wie beispielsweise Pyridin, bei Temperaturen zwischen 0 und 100°C, vorzugsweise zwischen 0 und 50°C umsetzt (vgl. auch die Herstellungsbeispiele).

Die Amide der Formel (VII) sind bekannt (vgl. z.B. EP-A 0 398 692) bzw. können sie nach den dort beschriebenen Verfahren in allgemein bekannter Art und Weise erhalten werden, indem man z.B. entsprechende Ester-Derivate (vgl. hierzu z.B. EP-A 0 253 213, EP-A 0 506 149, EP-A 0 463 488 oder EP-A 0 398 692) mit Ammoniak umsetzt (vgl. hierzu auch die Herstellungsbeispiele).

Ester-Derivate der allgemeinen Formel (IX) 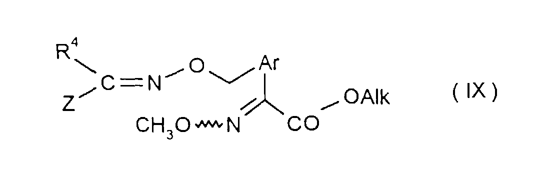 in welcher
- Ar, R⁴ und Z: die oben angegebene Bedeutung haben,
- Alk: für Alkyl, vorzugsweise C₁-C₄-Alkyl, wie insbesondere Methyl steht,
können auch erhalten werden, wenn man Ester-Derivate der allgemeinen Formel (X) (vgl. z.B. EP-A 0 506 149 und EP-A 0 600 835) 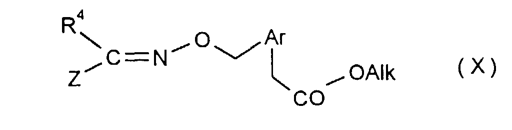 in welcher
- Ar, Alk, R⁴ und Z: die oben angegebene Bedeutung haben,
in bekannter Art und Weise mit einem Nitrosierungsmittel, wie beispielsweise tert.-Butylnitril in einem geeigneten Lösungsmittel, wie beispielsweise tert.-Butanol und in Gegenwart einer geeigneten Base, wie beispielsweise Kalium-tert.-butylat zunächst zu den entsprechenden Oxim-Derivaten der allgemeinen Formel (Xa) 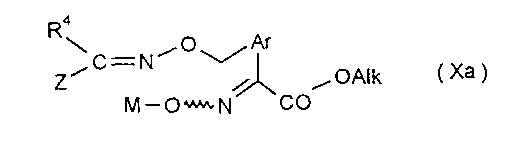 in welcher
- Ar, Alk, R⁴ und Z: die oben angegebene Bedeutung haben und
- M: für Wasserstoff oder ein Metalläquivalent, wie insbesondere Kalium oder Natrium steht,
umsetzt; und diese anschließend
entweder direkt in üblicher Art und Weise durch Alkylierung in die Ester-Derivate der Formel (IX) überführt,
oder nach Freisetzung und Isolierung der Oxime der Formel (Xa) mit M=Wasserstoff unter Verwendung einer Base, wie beispielsweise Kaliumcarbonat und eines Alkylierungsmittels, wie beispielsweise Dimethylsulfat in Gegenwart eines geeigneten Lösungsmittels, wie beispielsweise Acetonitril in die Ester-Derivate der Formel (IX) umwandelt (vgl. auch die Herstellungsbeispiele).

Die Ester-Derivate der allgemeinen Formel (X) können erhalten werden, indem man zunächst (1. Stufe) ein Oxim der allgemeinen Formel (XI) 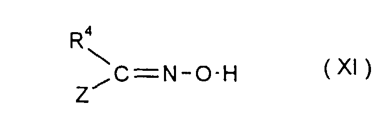 in welcher
- R⁴ und Z: die oben angegebene Bedeutung haben,
gegebenenfalls in Gegenwart eines Verdünnungsmittels, wie beispielsweise Methanol, Ethanol, Dimethylformamid oder N-Methylpyrrolidon bei Temperaturen zwischen -20°C und 200°C mittels einer Base, wie beispielsweise Alkalimetallalkoholate oder Metallhydride, insbesondere Natriumhydrid und Kaliumtert.-butanolat in das entsprechende Salz überführt und bei gleicher Temperatur mittels einem Dichlor-Derivat der allgemeinen Formel (XII) 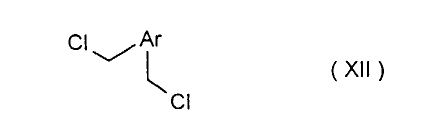 in welcher
- Ar: die oben angegebene Bedeutung hat,
zu einem O-Iminooxymethyl-Derivat der allgemeinen Formel (XIII) 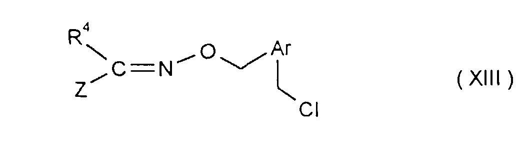 in welcher
- Ar, R⁴ und Z: die oben angegebene Bedeutung haben,
umsetzt, wobei das Dichlor-Derivat der Formel (XII) äquivalent, aber auch in beliebigem Überschuß, vorzugsweise von 1 bis 10 Äquivalenten eingesetzt werden kann;
anschließend (2. Stufe) die so erhaltenen O-Iminoxymethyl-Derivate der Formel (XIII) mit 1 bis 5, vorzugsweise 1 bis 1,1 Äquivalenten an Alkalimetallcyanid in Gegenwart eines Verdünnungsmittels, wie beispielsweise Aceton, Acetonitril, Methylehtylketon, Dimethylsulfoxid, Toluol, Methanol, Ethanol, Isopropanol, Butanol, Tetrahydrofuran, Dioxan oder Wasser bei Temperaturen zwischen 0°C und 100°C, vorzugsweise zwischen 20°C und 50°C, gegebenenfalls in Gegenwart eines Phasentransferkatalysators, wie beispielsweise Tetra-n-butylammoniumchlorid, Tetra-n-butylammoniumbromid, Tetra-n-butylammoniumhydrogensulfat, Tetramethylammoniumbromid oder Benzyltriethylammoniumchlorid zu einem Essigsäurenitril-Derivat der allgemeinen Formel (XIV) 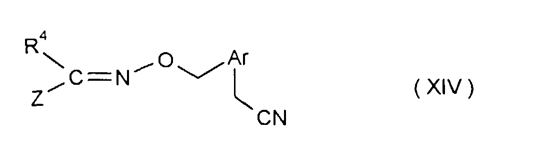 in welcher
- Ar, R⁴ und Z: die oben angegebene Bedeutung haben,
umsetzt;
und anschließend (3. Stufe) die so erhaltenen Essigsäurenitril-Derivate der Formel (XIV) in allgemein üblicher und bekannter Art und Weise
entweder direkt mittels Alkohol in Gegenwart einer wässrigen Säure zu den Ester-Derivaten der Formel (X) umsetzt;
oder zunächst mittels Hydrolyse in Gegenwart von starken Basen oder Mineralsäuren in die entsprechenden Essigsäure-Derivate der allgemeinen Formel (XV) 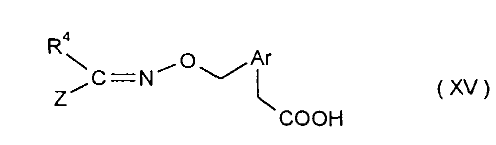 in welcher
- Ar, R⁴ und Z: die oben angegebene Bedeutung haben,
überführt und diese anschließend in allgemein üblicher und bekannter Art und Weise mittels Alkohol in Gegenwart einer Säure bzw. einer Base zu den Ester-Derivaten der Formel (X) umsetzt;
oder zunächst mittels eines Säurehalogenids, wie insbesondere PCl₃, PCl₅, POCl₃, SOCl₂ oder SO₂Cl₂ in die entsprechenden Essigsäurehalogenid-Derivate der allgemeinen Formel (XVI) 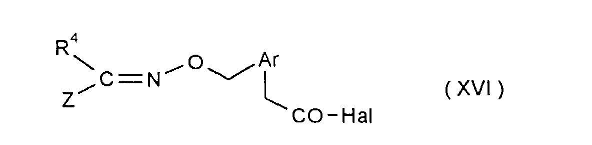 in welcher
- Ar, R⁴ und Z: die oben angegebene Bedeutung haben und
- Hal: für Halogen, vorzugsweise Chlor oder Brom, insbesondere Chlor steht,
überführt und diese anschließend in allgemein üblicher und bekannter Art und Weise mittels Alkohol, gegebenenfalls in Gegenwart eines Säurebindemittels zu den Ester-Derivaten der Formel (X) umsetzt.
(vgl. auch die Herstellungsbeispiele)

Die Oxime der Formel (XI) und die Dichlor-Derivate der Formel (XII) sind allgemein bekannte Verbindungen der organischen Chemie bzw. in allgemein üblicher Art und Weise herstellbar.

Die O-Iminooxymethyl-Derivate der Formel (XIII), die Essigsäurenitril-Derivate der Formel (XIV), die Essigsäure-Derivate der Formel (XV) und die Essigsäurehalogenid-Derivate der Formel (XVI) sind neu und ebenfalls Gegenstand dieser Erfindung.

Nitrile der Formel (IIa) 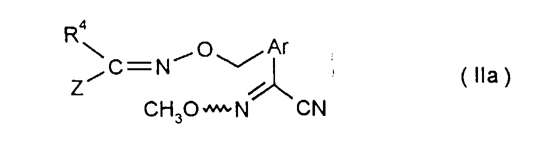 in welcher
- Ar, R⁴ und Z: die oben angegebene Bedeutung haben,
können auch erhalten werden, indem man 2-Methoximino-2-(2-brommethylphenyl)-acetonitril der Formel (XVII) 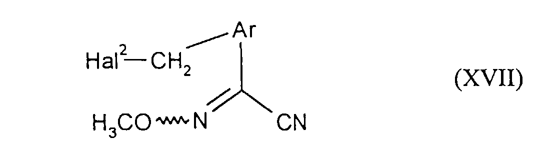 in welcher
- Hal²: für Halogen steht und
- Ar: die oben angegebene Bedeutung hat,
mit Oxim-Derivaten der allgemeinen Formel (XVIII) 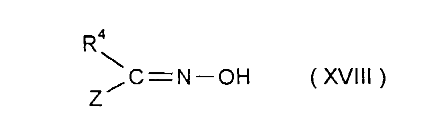 in welcher
- R⁴ und Z: die oben angegebene Bedeutung haben,
gegebenenfalls in Form ihrer Alkalimetallsalze in Gegenwart eines Verdünnungsmittels, wie beispielsweise Dimethylformamid und gegebenenfalls in Gegenwart einer Base, wie beispielsweise Natrium- oder Kaliumhydrid bei Temperaturen zwischen 0 und 100°C, vorzugsweise zwischen 0 und 80°C umsetzt (vgl. auch die Herstellungsbeispiele).

Die 2-Methoximinoacetonitrile der Formel (XVII) werden erhalten, indem in allgemein üblicher und bekannter Art und Weise zunächst 2-Methoximinoessigsäureester der Formel (XIX), 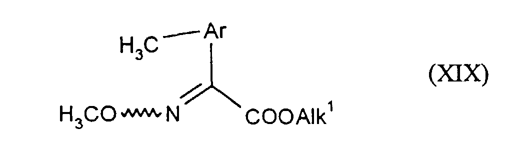 in welcher
- Ar: die oben angegebene Bedeutung hat und
- Alk¹: für Methyl oder Ethyl steht,
mittels Ammoniak in ein 2-Methoximinoacetamid der Formel (XX), 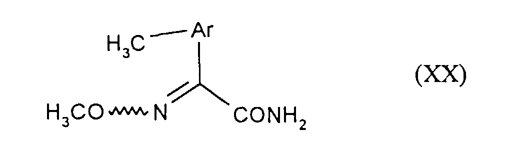 in welcher
- Ar: die oben angegebene Bedeutung hat,
überführt;

dieses mit wasserabspaltenden Mitteln, wie beispielsweise Anhydriden, gegebenenfalls in Gegenwart eines Reaktionshilfsmittels, wie beispielsweise Pyridin, bei Temperaturen zwischen 0 und 100°C vorzugsweise zwischen 0 und 50°C zu einem Nitrilderivat der Formel (XXI), 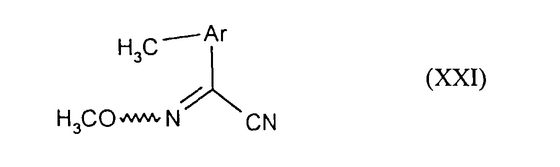 in welcher
- Ar: die oben angegebene Bedeutung hat,
umsetzt;
und schließlich das so erhaltene Nitril-Derivat der Formel (XXI) bromiert, beispielsweise durch Umsetzung mit N-Bromsuccinimid in Gegenwart eines Katalysators, wie beispielsweise Dibenzoylperoxid (vgl. auch die Herstellungsbeispiele).

Die Verbindungen der Formeln (XVII), (XX) und (XXI) sind neu und ebenfalls Gegenstand dieser Erfindung.

Die Oxim-Derivate der allgemeinen Formel (XVIII) sind bekannt (vgl. z.B. Houben-Weyl "Methoden der organischen Chemie" Band VII/I, S. 413-488) bzw. sind sie in allgemein bekannter Art und Weise erhältlich.

Der 2-Methoximinoessigsäuremethylester der Formel (XIX) sind bekannt und/oder nach bekannten Methoden herstellbar (vgl. z.B. EP-A 0 400 417).

Die außerdem zur Durchführung des erfindungsgemäßen Verfahrens (a) als Ausgangsstoffe benötigten (Thio) Alkohole sind duch die Formel (III) allgemein definiert. In der Formel (III) hat X¹ vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für X in seiner Bedeutung als Alkoxy bzw. Alkylthio genannt wurden.

Die (Thio) Alkohole der Formel (III) sowie deren Alkalimetallsalze, wie beispielsweise Natrium- und Kalium-Salze sind allgemein bekannte Verbindungen der organischen Chemie.

Die zur Durchführung der erfindungsgemäßen Verfahren (b), (c) und (f) als Ausgangsstoffe benötigten Imidoester sind durch die Formel (Ia) allgemein definiert. In der Formel (Ia) haben Ar, G, Z und X¹ (für X in seiner Bedeutung als Alkoxy bzw. Alkylthio) vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diese Substituenten genannt wurden.

Die Imidoester der Formel (Ia) sind erfindungsgemäße Verbindungen und gemäß Verfahren (a) erhältlich.

Imidoester der Formel (Ia), in denen X¹ für Alkylthio steht, können auch erhalten werden, indem man Amide der allgemeinen Formel (VII) mit einem Schwefelungsmittel, wie z.B. P₄S₁₀ oder Lawesson-Reagenz, gegebenenfalls in einem Verdünnungsmittel, wie z.B. Xylol oder Toluol, bei Temperaturen zwischen 50°C und 200°C umsetzt und die so erhaltenen Thioamide der allgemeinen Formel (VIII) 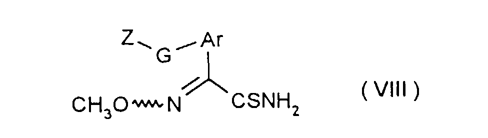 in welcher
- Ar, G und Z: die oben angegebene Bedeutung haben,
in üblicher Art und Weise alkyliert.

Die außerdem zur Durchführung des erfindungsgemäßen Verfahrens (b) als Ausgangsstoffe benötigten Säurehalogenide sind durch die Formel (IV) allgemein definiert. In der Formel (IV) hat R vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diesen Substituenten genannt wurden. Hal steht vorzugsweise für Fluor, Chlor oder Brom, besonders bevorzugt für Chlor.

Die Säurehalogenide der Formel (IV) sind allgemein bekannte Verbindungen der organischen Chemie.

Das außerdem zur Durchführung des erfindungsgemäßen Verfahrens (c) als Ausgangsstoff benötigte Cyanamid der Formel (V) ist ebenfalls eine allgemein bekannte Verbindung der organischen Chemie.

Die zur Durchführung des erfindungsgemäßen Verfahrens (d) als Ausgangsstoffe benötigten Imidsäurederivate sind durch die Formel (Ib) allgemein definiert. In der Formel (Ib) haben Ar, G, Z, X¹ (für X in seiner Bedeutung als Alkoxy bzw. Alkylthio) und R' (für R in seiner oben genannten Bedeutung mit Ausnahme von Wasserstoff) vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diese Substituenten genannt wurden.

Die Imidsäurederivate der Formel (Ib) sind erfindungsgemäße Verbindungen und gemäß den Verfahren (b) und (c) erhältlich.

Die außerdem zur Durchführung der erfindungsgemäßen Verfahren (d) und (f) als Ausgangsstoffe benötigten Amine sind durch die Formel (VI) allgemein definiert. In der Formel (VI) hat X² vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für X in seiner Bedeutung als Amino, Alkylamino und Dialkylamino genannt wurden.

Die Amine der Formel (VI) sind allgemein bekannte Verbindungen der organischen Chemie.

Die zur Durchführung des erfindungsgemäßen Verfahrens (e) als Ausgangsstoffe benötigten Imidasäurederivate sind durch die Formel (Ic) allgemein definiert. In der Formel (Ic) haben Ar, G, Z und R' (für R in seiner oben genannten Bedeutung mit Ausnahme von Wasserstoff) vorzugsweise bzw. besonders bevorzugt diejenigen Bedeutungen, die bereits oben im Zusammenhang mit der Beschreibung der Verbindungen der Formel (I) als bevorzugt bzw. besonders bevorzugt für diese Substituenten genannt wurden.

Die Imidsäurederivate der Formel (Ic) sind erfindungsgemäße Verbindungen und gemäß den Verfahren (b) und (c) erhältlich.

Das außerdem zur Durchführung des erfindungsgemäßen Verfahrens (e) als Ausgangsstoff benötigte Hydrazin bzw. Hydrahinhydrat ist eine allgemein bekannte Verbindung der organischen Chemie.

Als Verdünnungsmittel kommen bei der Durchführung des erfindungsgemäßen Verfahrens (a) alle für derartige Umsetzungen üblichen, inerten organischen Lösungsmittel infrage. Vorzugsweise verwendbar sind Ether, wie Diethylether, Methyl-tert.-butyl-ether, Dioxan, Tetrahydrofuran oder Ethylenglykoldimethyl- oder -diethylether; ferner Nitrile, wie Acetonitril; sowie außerdem gegebenenfalls halogenierte aliphatische, cycloaliphatische und aromatische Kohlenwasserstoffe, wie Methylenchlorid, Chloroform, Tetrachlorkohlenstoff, Hexan, Cyclohexan, Benzol, Toluol, Xylol, Chlorbenzol und Petrolether.

Das erfindungsgemäße Verfahren (a) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -alkoholate,-acetate, -carbonate oder -hydrogencarbonate, wie beispielsweise Natriumhydroxid, Natriummethylat, Natriumethylat, Kalium-t-butylat, Natriumacetat, Kaliumacetat, Natriumcarbonat, Kaliumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU). Auch saure Reaktionshilfsmittel wie beispielsweise p-Toluolsulfonsäure sind gegebenenfalls von Vorteil.

Die Reaktionstemperaturen können bei der Durchführung des erfindungsgemäßen Verfahrens (a) in einem größeren Bereich variiert werden. Im allgemeinen arbeitet man bei Temperaturen zwischen 0°C und 200°C, vorzugsweise bei Temperaturen zwischen 0°C und 100°C.

Zur Durchführung des erfindungsgemäßen Verfahrens (a) setzt man pro Mol an Nitril der Formel (II) im allgemeinen 1 bis 10 Mol, vorzugsweise 1 bis 5 Mol an (Thio) Alkohol der Formel (III) bzw. deren Alkalimetallsalze und gegebenenfalls 1 bis 10 Mol, vorzugsweise 1 bis 5 Mol an Reaktionshilfsmittel ein. Es ist jedoch auch ein größerer Überschuß möglich.

Als Verdünnungsmittel zur Durchführung des erfindungsgemäßen Verfahrens (b) kommen inerte organische Lösungsmittel infrage. Vorzugsweise verwendbar sind aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff; Ether, wie Diethylether, Dioxan, Tetrahydrofuran oder Ethylenglykoldimethyl- oder -diethylether oder Ester, wie Essigsäureethylester.

Das erfindungsgemäße Verfahren (b) wird vorzugsweise in Gegenwart eines geeigneten Reaktionshilfsmittels durchgeführt. Als solche kommen alle üblicherweise verwendbaren anorganischen und organischen Basen in Frage. Vorzugsweise verwendet man Alkalimetallhydride, -hydroxide, -alkoholate,-acetate, -carbonate oder -hydrogencarbonate, wie beispielsweise Natriumhydroxid, Natriummethylat, Natriumethylat, Kalium-t-butylat, Natriumacetat, Kaliumacetat, Natriumcarbonat, Kaliumcarbonat oder Natriumhydrogencarbonat oder auch tertiäre Amine, wie beispielsweise Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0°C und 150°C, vorzugsweise bei Temperaturen zwischen 0°C und 100°C.

Zur Durchführung des erfindungsgemäßen Verfahrens (b) setzt man pro Mol an Imidoester der Formel (Ia) im allgemeinen 0,8 bis 5 Mol, vorzugsweise 0,8 bis 3 Mol an Säurederivat der Formel (IV) und gegebenenfalls 1 bis 5 Mol, vorzugsweise 1 bis 3 Mol an Reaktionshilfsmittel ein.

Als Verdünnungsmittel zur Durchführung des erfindungsgemäßen Verfahrens (c) kommen Wasser sowie alle mit Wasser mischbaren organischen Lösungsmittel infrage einschließlich deren Mischungen mit Wasser. Vorzugsweise genannt seien Alkohole, wie z.B. Methanol oder Ethanol.

Das erfindungsgemäße Verfahren (c) wird vorzugsweise in Gegenwart eines geeigneten Reaktionshilfsmittels durchgeführt. Als solche kommen vorzugsweise anorganische Salze, wie Alkalimetallacetate, -carbonate, -hydrogencarbonate,-phosphate, -hydrogenphosphate oder -dihydrogenphosphate infrage, wie beispielsweise Natriumacetat, Kaliumacetat, Natriumcarbonat, Kaliumcarbonat, Natriumhydrogencarbonat, Natriumphosphat, Natriumdihydrogenphosphat und Dinatriumhydrogenphosphat.

Die Reaktionstemperaturen können bei der Durchführung des erfindungsgemäßen Verfahrens (c) in einem größeren Bereich variiert werden. Im allgemeinen arbeitet man bei Temperaturen zwischen -20°C und +100°C, vorzugsweise bei Temperaturen zwischen 0°C und 50°C.

Zur Durchführung des erfindungsgemäßen Verfahrens (c) setzt man pro Mol an Imidoester der Formel (Ia) im allgemeinen 0,5 bis 5 Mol, vorzugsweise 0,5 bis 2 Mol an Cyanamid der Formel (V) und gegebenenfalls 2 bis 20 Mol, vorzugsweise 2 bis 8 Mol an Reaktionshilfsmittel ein, wobei der pH-Wert der Reaktionsmischung im allgemeinen zwischen 4 und 9, vorzugsweise zwischen 6 und 7 liegen sollte.

Als Verdünnungsmittel zur Durchführung der erfindungsgemäßen Verfahren (d) und (e) kommen Wasser sowie alle mit Wasser mischbaren inerten organischen Lösungsmittel infrage. Vorzugsweise verwendbar sind Ether, wie Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäureamid oder Sulfoxide, wie Dimethylsulfoxid, Alkohole, wie Methanol oder Ethanol oder basische Lösungsmittel wie Pyridin oder Triethylamin.

Die Reaktonstemperaturen können bei der Durchführung der erfindungsgemäßen Verfahren (d) und (e) in einem größeren Bereich variiert werden. Im allgemeinen arbeitet man bei Temperaturen zwischen -50°C und +100°C, vorzugsweise bei Temperaturen zwischen -20°C und +50°C.

Zur Durchführung des erfindungsgemäßen Verfahrens (d) arbeitet man vorzugsweise in molaren Mengen; es ist aber auch möglich, das Amin der Formel (VI) in größerem Überschuß einzusetzen.

Zur Durchführung des erfindungsgemäßen Verfahrens (e) setzt man pro Mol an Imidsäurederivat der Formel (Ic) im allgemeinen 0,5 bis 10 Mol, vorzugsweise 0,5 bis 5 Mol an Hydrazin bzw. Hydrazinhydrat ein.

Als Verdünnungsmittel zur Durchführung des erfindungsgemäßen Verfahrens (f) kommen Wasser sowie polare organische Lösungsmittel infrage. Vorzugsweise verwendbar sind Ether, wie Diethylether, Dioxan, Tetrahydrofuran oder Ethylenglykoldimethyl- oder -diethylether sowie Alkohole, wie Methanol oder Ethanol. Es ist auch möglich, das als Reaktionspartner einzusetzende Amin der Formel (VI) zu verwenden.

Die Reaktionstemperaturen können bei der Durchführung des erfindungsgemäßen Verfahrens (f) in einem größeren Bereich variiert werden. Im allgemeinen arbeitet man bei Temperaturen zwischen -40°C und +200°C, vorzugsweise bei Temperaturen zwischen 0°C und 100°C.

Zur Durchführung des erfindungsgemäßen Verfahrens (f) arbeitet man vorzugsweise in molaren Mengen; es ist aber auch möglich, insbesondere das Amin der Formel (VI) in größerem Überschuß einzusetzen.

Bei der Durchführung der erfindungsgemäßen Verfahren (a) bis (f) arbeitet man im allgemeinen unter Normaldruck. Es ist jedoch auch möglich, unter vermindertem oder insbesondere und erhöhtem Druck zu arbeiten.

Die Reaktionsdurchführung, Aufarbeitung und Isolierung der Reaktionsprodukte erfolgt nach allgemein üblichen Methoden (vgl. auch die Herstellungsbeispiele).

Die erfindungsgemäßen Wirkstoffe der Formel (I) weisen eine starke mikrobizide Wirkung auf und können zur Bekämpfung von unerwünschten Mikroorganismen praktisch eingesetzt werden. Die Wirkstoffe sind für den Gebrauch als Pflanzenschutzmittel, insbesondere als Fungizide geeignet.

Fungizide Mittel im Pflanzenschutz werden eingesetzt zur Bekämpfung von Plasmoiophoromycetes, Oomycetes, Chytridiomycetes, Zygomycetes, Ascomycetes, Basisdiomycetes, Deuteromycetes.

Beispielhaft aber nicht begrenzend seien einige Erreger von pilzlichen Krankheiten, die unter die oben aufgezählten Oberbegriffe fallen, genannt:
Pythium-Arten, wie beispielsweise Pythium ultimum;
Phytophthora-Arten, wie beispielsweise Phytophthora infestans;
Pseudoperonospora-Arten, wie beispielsweise Pseudoperonospora humuli oder Pseudoperonospora cubense;
Plasmopara-Arten, wie beispielsweise Plasmopara viticola;
Peronospora-Arten, wie beispielsweise Peronospora pisi oder Peronospora brassicae;
Erysiphe-Arten, wie beispielsweise Erysiphe graminis;
Sphaerotheca-Arten, wie beispielsweise Sphaerotheca fuliginea;
Podosphaera-Arten, wie beispielsweise Podosphaera leucotricha;
Venturia-Arten, wie beispielsweise Venturia inaequalis;
Pyrenophora-Arten, wie beispielweise Pyrenophora teres oder Pyrenophora graminea (Konidienform: Drechslera, Synonym: Helminthosporium);
Cochliobolus-Arten, wie beispielsweise Cochliobolus sativus (Konidienform:
Drechslera, Synonym: Helminthosporium);
Uromyces-Arten, wie beispielsweise Uromyces appendiculatus;
Puccinia-Arten, wie beispielsweise Puccinia recondita;
Tilletia-Arten, wie beispielsweise Tilletia caries;
Ustilago-Arten, wie beispielsweise Ustilago nuda oder Ustilago avenae;
Pellicularia-Arten, wie beispielsweise Pellicularia sasakii;
Pyricularia-Arten, wie beispielsweise Pyricularia oryzae;
Fusarium-Arten, wie beispielsweise Fusarium culmorum;
Botrytis-Arten, wie beispielsweise Botrytis cinerea;
Septoria-Arten, wie beispielsweise Septoria nodorum;
Leptosphaeria-Arten, wie beispielsweise Leptosphaeria nodorum;
Cercospora-Arten, wie beispielsweise Cercospora canescens;
Alternaria-Arten, wie beispielsweise Alternaria brassicae;
Pseudocercosporella-Arten, wie beispielsweise Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Dabei können die erfindungsgemäßen Wirkstoffe mit besonders gutem Erfolg zur Bekämpfung von Krankheiten im Obst- und Gemüseanbau, wie beispielsweise gegen Podosphaera- und Venturia-Arten, zur Bekämpfung von Getreidekrankheiten, wie beispielsweise gegen Erysiphe-Arten, oder zur Bekämpfung von Reiskrankheiten, wie beispielsweise gegen Pyricularia oryzae eingesetzt werden.

Die Wirkstoffe können in Abhängigkeit von ihren jeweiligen physikalischen und/oder chemischen Eigenschaften in übliche Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Kalt- und -Warmnebel-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen infrage: Aromaten, wie Xylol, Toluol, Alkylnaphthaline, chlorierte Aromaten oder chlorierte aliphatische Kohlenwasserstoffe, wie Chlorbenzole, Chlorethylene, oder Methylenchlorid, aliphatsche Kohlenwasserstoffe, wie Cyclohexan oder Paraffine, z.B. Erdölfraktionen, Alkohole, wie Butanol oder Glykol sowie deren Ether und Ester, Ketone, wie Aceton, Methylethylketon, Methylisobutylketon oder Cyclohexanon, stark polare Lösungsmittel, wie Dimethylformamid oder Dimethylsulfoxid, sowie Wasser; mit verflüssigten gasförmigen Streckmitteln oder Trägerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen infrage: z.B. natürliche Gesteinsmehle, wie Kaoline, Tonerden, Talkum, Kreide, Quartz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier- und/oder schaumerzeugende Mittel kommen infrage: z.B. nicht ionogene und anionische Emulgatoren, wie Polyoxyethylen-Fettsäureester, Polyoxyethylen-Fettalkohol-Ether, z.B. Alkylarylpolyglykolether, Alkylsulfonate, Alkylsulfate, Arylsulfonate sowie Eiweißhydrolysate; als Dispergiermittel kommen infrage: z.B. Ligninsulfitablaugen und Methylcellulose.

Es können in den Formulierungen Haftmittel wie Carboxymethylcellulose, natürliche und synthetische, pulverige, körnige oder latexförmige Polymere verwendet werden, wie Gummiarabicum, Polyvinylalkohol, Polyvinylacetat, sowie natürliche Phospholipide, wie Kephaline und Lecithine und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinenzwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Fungiziden, Bakteriziden, Akariziden, Nematiziden oder Insektiziden verwendet werden, um so z.B. das Wirkungsspektrum zu verbreitern oder Resistenzentwicklungen vorzubeugen.

Für die Mischungen kommen beispielsweise infrage:

### Fungizide:

2-Aminobutan; 2-Anilino-4-methyl-6-cyclopropyl-pyrimidin; 2',6'-Dibromo-2-methyl-4'-trifluoromethoxy-4'-trifluoro-methyl-1,3-thizole-5-carboxanilid; 2,6-Dichloro-N-(4-trifluoromethylbenzyl)benzamid; (E)-2-Methoxyimino-N-methyl-2-(2-phenoxyphenyl) acetamid; 8-Hydroxyquinolinsulfat; Methyl-(E)-2-{2-[6-(2-cyano-phenoxy)pyrimidin-4-yloxy]phenyl}-3-methoxyacrylat; Methyl-(E)-methoximino [alpha-(o-tolyloxy)-o-tolyl] acetat; 2-Phenylphenol (OPP), Aldimorph, Ampropylfos, Anilazin, Azaconazol,
Benalaxyl, Benodanil, Benomyl, Binapacryl, Biphenyl, Bitertanol, Blasticidin-S, Bromuconazole, Bupirimate, Buthiobate,
Calciumpolysulfid, Captafol, Captan, Carbendazim, Carboxin, Chinomethionat (Quinomethionat), Chloroneb, Chloropicrin, Chlorothalonil, Chlozolinat, Cufraneb, Cymoxanil, Cyproconazole, Cyprofuram,
Dichlorophen, Diclobutrazol, Diclofluanid, Diclomezin, Dicloran, Diethofencarb, Difenoconazol, Dimethirimol, Dimethomorph, Diniconazol, Dinocap, Diphenylamin, Dipyrithion, Ditalimfos, Dithianon, Dodin, Drazoxolon,
Edifenphos, Epoxyconazole, Ethirimol, Etridiazol,
Fenarimol, Fenbuconazole, Fenfuram, Fenitropan, Fenpiclonil, Fenpropidin, Fenpropi-morph, Fentinacetate, Fentinhydroxyd, Ferbam, Ferimzone, Fluazinam, Fludioxonil, Fluoromide, Fluquinconazole, Flusilazole, Flusulfamide, Flutolanil, Flutriafol, Folpet, Fosetyl-Aluminium, Fthalide, Fuberidazol, Furalaxyl, Furmecyclox,
Guazatine,
Hexachlorobenzol, Hexaconazol, Hymexazol,
Imazalil, Imibenconazol, Iminoctadin, Iprobenfos (IBP), Iprodion, Isoprothiolan, Kasugamycin, Kupfer-Zubereitungen, wie: Kupferhydroxid, Kupfernaphthenat, Kupferoxychlorid, Kupfersulfat, Kupferoxid, Oxin-Kupfer and Bordeaux-Mischung,
Mancopper, Mancozeb, Maneb, Mepanipyrim, Mepronil, Metalaxyl, Metconazol, Methasulfocarb, Methfuroxam, Metiram, Metsulfovax, Myclobutanil,
Nickel dimethyldithiocarbamat, Nitrothal-isopropyl, Nuarimol,
Ofurace, Oxadixyl, Oxamocarb, Oxycarboxin,
Pefurazoat, Penconazol, Pencycuron, Phosdiphen, Pimaricin, Piperalin, Polyoxin, Probenazol, Prochloraz, Procymidon, Propamocarb, Propiconazole, Propineb, Pyrazophos, Pyrifenox, Pyrimethanil, Pyroquilon,
Quintozen (PCNB),
Schwefel und Schwefel-Zubereitungen,
Tebuconazol, Tecloftalam, Tecnazen, Tetraconazol, Thiabendazol, Thicyofen, Thiophanat-methyl, Thiram, Tolclophos-methyl, Tolylfluanid, Triadimefon, Triadimenol, Triazoxid, Trichlamid, Tricyclazol, Tridemorph, Triflumizol, Triforin, Triticonazol,
Validamycin A, Vinclozolin,
Zineb, Ziram

### Bakterizide:

Bronopol, Dichlorophen, Nitrapyrin, Nickel Dimethyldithiocarbamat, Kasugamycin, Octhilinon, Furancarbonsäure, Oxytetracyclin, Probenazol, Streptomycin, Tecloftalam, Kupfersulfat und andere Kupfer-Zubereitungen.

### Insektizide / Akarizide / Nematizide:

Abamectin, Abamectin, AC 303 630, Acephat, Acrinathrin, Alanycarb, Aldicarb, Alphamethrin, Amitraz, Avermectin, AZ 60541, Azadirachtin, Azinphos A, Azinphos M, Azocyclotin,
Bacillus thuringiensis, Bendiocarb, Benfuracarb, Bensultap, Betacyluthrin, Bifenthrin, BPMC, Brofenprox, Bromophos A, Bufencarb, Buprofezin, Butocarboxin, Butylpyridaben,
Cadusafos, Carbaryl, Carbofuran, Carbophenothion, Carbosulfan, Cartap, CGA 157 419, CGA 184699, Chloethocarb, Chlorethoxyfos, Chloretoxyfos, Chlorfenvinphos, Chlorfluazuron, Chlormephos, Chlorpyrifos, Chlorpyrifos M, Cis-Resmethrin, Clocythrin, Clofentezin, Cyanophos, Cycloprothrin, Cyfluthrin, Cyhalothrin, Cyhexatin, Cypermethrin, Cyromazin,
Deltamethrin, Demeton M, Demeton S, Demeton-S-methyl, Diafenthiuron, Diazinon, Dichlofenthion, Dichlorvos, Dicliphos, Dicrotophos, Diethion, Diflubenzuron, Dimethoat, Dimethylvinphos, Dioxathion, Disulfoton,
Edifenphos, Emamectin, Esfenvalerat, Ethiofencarb, Ethion, Ethofenprox, Ethoprophos, Etofenprox, Etrimphos,
Fenamiphos, Fenazaquin, Fenbutatinoxid, Fenitrothion, Fenobucarb, Fenothiocarb, Fenoxycarb, Fenpropathrin, Fenpyrad, Fenpyroximat, Fenthion, Fenvalerate, Fipronil, Fluazinam, Flucycloxuron, Flucythrinat, Flufenoxuron, Flufenprox, Fluvalinate, Fonophos, Formothion, Fosthiazat, Fubfenprox, Furathiocarb,
HCH, Heptenophos, Hexaflumuron, Hexythiazox,
Imidacloprid, Iprobenfos, Isazophos, Isofenphos, Isoprocarb, Isoxathion, Ivemectin,
Lamda-cyhalothrin, Lufenuron,
Malathion, Mecarbam, Mervinphos, Mesulfenphos, Metaldehyd, Methacrifos, Methamidophos, Methidathion, Methiocarb, Methomyl, Metolcarb, Milbemectin, Monocrotophos, Moxidectin,
Naled, NC 184, NI 25, Nitenpyram
Omethoat, Oxamyl, Oxydemethon M, Oxydeprofos,
Parathion A, Parathion M, Permethrin, Phenthoat, Phorat, Phosalon, Phosmet, Phosphamdon, Phoxim, Pirimicarb, Pirimiphos M, ,Primiphos A, Profenofos, Profenophos, Promecarb, Propaphos, Propoxur, Prothiofos, Prothiophos, Prothoat,
Pymetrozin, Pyrachlophos, Pyraclofos, Pyraclophos, Pyradaphenthion, Pyresmethrin, Pyrethrum, Pyridaben, Pyrimidifen, Pyriproxifen,
Quinalphos,
RH 5992,
Salithion, Sebufos, Silafluofen, Sulfotep, Sulprofos,
Tebufenozid, Tebufenpyrad, Tebupirimphos, Teflubenzuron, Tefluthrin, Temephos, Terbam, Terbufos, Tetrachlorvinphos, Thiafenox, Thiodicarb, Thiofanox, Thiomethon, Thionazin, Thuringiensin, Tralomethrin, Triarathen, Triazophos, Triazuron, Trichlorfon, Triflumuron, Trimethacarb,
Vamidothion, XMC, Xylylcarb, YI 5301 / 5302, Zetamethrin.

Auch eine Mischung mit anderen bekannten Wirkstoffen, wie Herbiziden oder mit Düngemitteln und Wachstumsregulatoren ist möglich.

Die Wirkstoffe können als solche, in Form ihrer Formulierungen oder den daraus bereiteten Anwendungsformen, wie gebrauchsfertige Lösungen, Suspensionen, Spritzpulver, Pasten, lösliche Pulver, Stäubemittel und Granulate angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Bei der Behandlung von Pflanzenteilen können die Wirkstoffkonzentrationen in den Anwendungsformen in einem größeren Bereich variiert werden: Sie liegen im allgemeinen zwischen 1 und 0,0001 Gew.-%, vorzugsweise zwischen 0,5 und 0,001Gew.-%.

Bei der Saatgutbehandlung werden im allgemeinen Wirkstoffmengen von 0,001 bis 50 g je Kilogramm Saatgut, vorzugsweise 0,01 bis 10 g benötigt.

Bei der Behandlung des Bodens sind Wirkstoffkonzentrationen von 0,00001 bis 0,1 Gew.-%, vorzugsweise von 0,0001 bis 0,02 Gew.-% am Wirkungsort erforderlich.

### Herstellungsbeispiele

### Beispiel 1

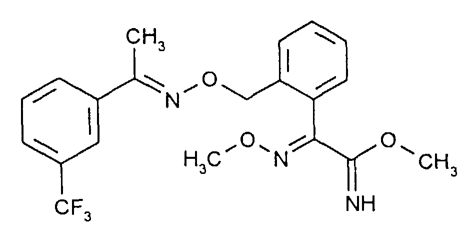

### (Verfahren a)

Eine Lösung von 11,3 g (0,03 mol) E-2-Methoximino-2-{2-[1-(3-trifluormethylphenyl)-ethylideniminoxymethyl]-phenyl}-acetonitril und 13,0 g (0,24 mol) Natriummethanolat in 46 ml Methanol wird 48 Stunden bei Raumtemperatur gerührt. Die Lösung wird auf 3 1 Wasser gegossen, mit Ether extrahiert, mit Kaliumcarbonat getrocknet und eingeengt. Man erhält 12,2 g eines gelblichen Öls mit einem Gehalt von 80 % (GC) E-2-Methoximino-2-{2-[l-(3-trifluormethylphenyl)-ethylideniminoxymethyl]-phenyl)-acetimidsäuremethylester.
¹H-NMR (CDCl3): d = 2,22; 3,86: 3,99; 5,11: 7,11-7,95.

### Herstellung des Ausgansproduktes

### (Variante 1)

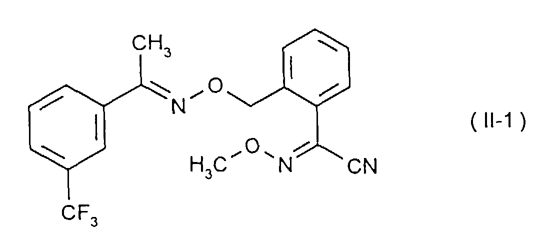

Eine Lösung von 2,8 g (0,007 mol) E-2-Methoximino-2-{2-[1-(3-trifluormethylphenyl)-ethylideniminoxymethyl]-phenyl}-acetamid in 5 ml Pyridin wird auf 0°C gekühlt, mit 2,2 g (0,0105 mol) Triluoracetanhydrid versetzt und 2 Stunden bei 0°C und 18 Stunden bei Raumtemperatur gerührt. Die Lösung wird eingeengt, der Rückstand in Essigester aufgenommen, mit Wasser und stark verdünnter Salzsäure gewaschen, mit Magnesiumsulfat getrocknet und eingeengt. Der Rückstand wird mit Cyclohexan/Essigester (3:1) an Kieselgel chromatografiert. Man erhält 1,8 g (69%) E-2-Methoximino-2-{2-[1-(3-trifluormethylphenyl)-ethylideniminoxymethyl]-phenyl}-acetonitril als gelbes Öl (IR-Spektrum: 2200 cm⁻¹).

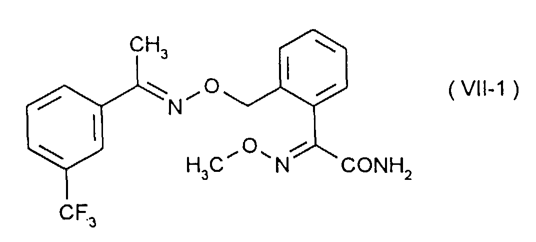

In eine Lösung von 32,6 g (0,08 mol) E-2-Methoximino-2-(2-[l-(3-trifluormethylphenyl)-ethylideniminoxymethyl]-phenyl}-essigsäuremethylester in 200 ml Methanol wird bei ca. 10 °C bis zur Sättigung Ammoniak eingeleitet. Es wird noch 2 Stunden gerührt und danach eingeengt. Der Rückstand wird mit Hexan verrührt und abgesaugt. Man erhält 23,8 g (76 %) E-2-Methoximino-2-{2-[1-(3-trifluormethylphenyl)-ethylideniminoxymethyl]-phenyl}-acetamid vom Schmelzpunkt 112 °C.

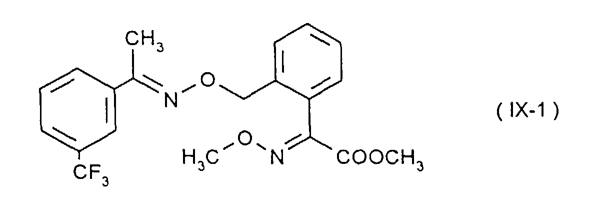

Zu einer Suspension von 4,5 g (0,113 mol) Natriumhydrid in 80 ml Dimethylformamid gibt man 19,1 g (0,094 mol) 3-Trifluormethylacetophenonoxim und rührt bis zum Ende der Gasentwicklung. Nun gibt man 26,9 g (0,094 mol) E-2-Methoximino-2-(2-brommethylphenyl)-essigsäuremethylester zu und rührt über Nacht bei Raumtemperatur. Das Lösungsmittel wird abdestilliert, der Rückstand auf 1 1 Wasser gegossen und mit Ether extrahiert. Die organische Phase wird mit Magnesiumsulfat getrocknet und eingeengt. Man erhält 34,2 g (89 %) E-2-Methoximino-2-{2-[1-(3-trifluormethylphenyl)-ethylideniminoxymethyl]-phenyl}-essigsäuremethylester als braunes Öl.

### Alternativherstellung der Verbindung (IX-1)

### 1. Stufe

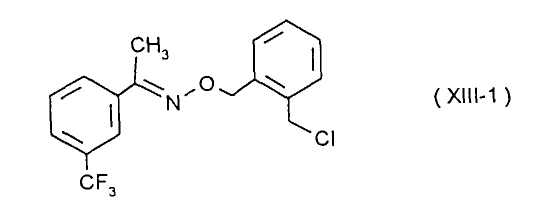

6,1 g (0,03 Mol) 3-Trifluormethylacetophenonoxim werden in 30 ml Dimethylformamid gelöst und durch Zugabe von 1,2 g (0,03 Mol) 60 %-igem Natriumhydrid bei Raumtemperatur durch ca. 30 minütiges Rühren ins Salz übergeführt. Diese Salzlösung tropft man zu 21 g (0,12 Mol) α,α-Dichlorxylol, welches man in 50 ml Dimethylformamid gelöst hat. Man erwärmt das Reaktionsgemisch 1 Stunde auf 50°C und destilliert das Gemisch im Hochvakuum. Nachdem Dimethylformamid und überschüssiges α,α-Dichlorxylol abdestilliert sind, gehen bei 0,3 Torr und 151 bis 156°C 8,4 g (81,9 % der Theorie) 2-[1-(3-Trifluormethylphenyl)-ethylideniminoxymethyl]-benzylchlorid über.
¹H-NMR-Spektrum (CDCl₃/TMS):
- δ =: 2,249 (3H); 4,751 (2H); 5,393 (2H); 7,299-7,39 (2H); 7,39-7,462 (3H); 7,563/7,589 (1H); 7,784/7,811 (1H); 7,895 (1H) ppm.

### 2. Stufe

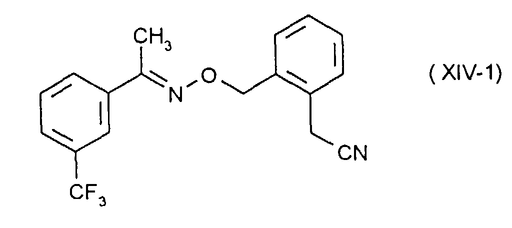

63,8 g (0,187 Mol) 2-[1-(3-Trifluormethylphenyl)-ethylideniminoxymethyl]-benzylchlorid werden zu 10,1 g (0,206 Mol) Natriumcyanid in 200 ml Dimethylsulfoxid gegeben und 12 Stunden bei Raumtemperatur gerührt Man gießt auf Wasser, extrahiert mit Essigsäureethylester, engt die organische Phase ein und verrührt den Rücksstand mit tiefsiedendem Petrolether. Man erhält 43 g (69,2 % der Theorie) 2-[1-(3-Trifluormethylphenyl)-ethylideniminoxymethyl]-phenylessigsäurenitril. ¹H-NMR-Spektrum (CDCl₃/TMS):
- δ =: 2,263 (3H); 3,899 (2H); 5,278 (2H); 7,249-7,506 (5H); 7,597/7,623 (1H); 7,788/7,814 (1H); 7,885 (1H) ppm.

### 3. Stufe

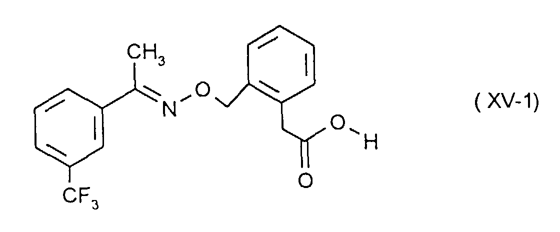

15 g (0,045 Mol) 2-[1-(3-Trifluormethylphenyl)-ethylideniminoxymethyl]-phenylessigsäurenitril werden in 90 ml Ethylenglykol mit 7 g (0,106 Mol) 85 %-igem KOH-Pulver 7 Stunden bei 140°C gerührt. Man gießt auf Wasser, extrahiert mit Essigsäureethylester. Dann säuert man an und extrahiert das Produkt mit Dichlormethan. Man erhält nach dem Abziehen des Lösungsmittels 13 g (82,2 % der Theorie) Säure.
¹H-NMR-Spektrum (CDCl₃/TMS):
- δ =: 2,181 (3H); 3,826 (2H); 5,291 (2H); 7,248-7,339 (3H); 7,421-7,468 (2H); 7,570/7,596 (1H); 7,761/7,787 (1H); 7,858 (1H) ppm.

### 4. Stufe

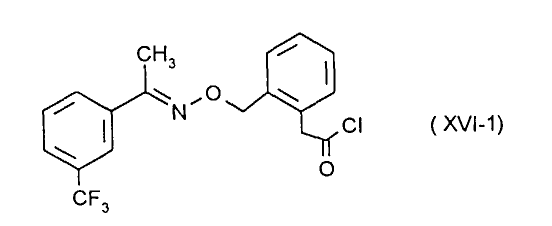

3,5 g (0,05 Mol) 2-[1-(3-Trifluormethylphenyl)-ethylideniminoxymethyl]-phenylessigsäure werden in 10 ml Dichlorethan mit 1,4 g (0,012 Mol) Thionylchlorid 2 Stunden lang am Rückfluß gekocht. Man erhält nach dem Abziehen der flüchtigen Bestandteile 3,5 g (94,7 % der Theorie) rohes 2-[1-(3-Trifluormethylphenyl)-ethylideniminoxymethyl]-phenylessigsäurechlorid.
¹H-NMR-Spektrum (CDCl₃/TMS):
- δ =: 2,236 (3H); 4,334 (2H); 5,268 (2H); 7,249-7,291 (1H); 7,357-7,387 (2H); 7,436-7,501 (2H); 7,593/7,619 (1H); 7,793/7,819 (1H); 7,896 (1H) ppm.

### 5. Stufe

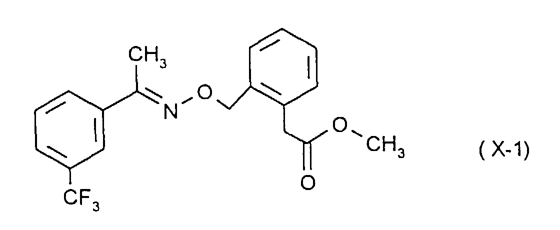

2,4 g (6,5 mMol) 2-[1-(3-Trifluormethylphenyl)-ethylideniminoxymethyl]-phenylessigsäurechlorid werden unter Kühlung bei 25°C zu einer Mischung aus 10 ml Methanol und 0,7 g (6,9 mMol) Triethylamin gegeben. Nach 10 Minuten bei Raumtemperatur engt man ein und verteilt den Rückstand zwischen Essigsäureethylester und Wasser. Nach dem Abziehen des Lösungsmittels erhält man 2 g (84,3 % der Theorie) 2-[1-(3-Trifluormethylphenyl)-ethylideniminoxymethyl]-phenylessigsäuremethylester. Das rohe Produkt besitzt nach GC/MS-Analyse einen Gehalt von 96,1 %.
¹H-NMR-Spektrum (CDCl₃/TMS):
- δ =: 2,238 (3H); 3,677(3H); 3,817 (2H); 5,307 (2H); 7,241-7,33 (3H); 7,42-7,49 (2H); 7,581/7,607 (1H); 7,798/7,824 (1H); 7,899 (1H) ppm.
GC/MS-Analyse:
Retentionsindex = 2198
M = 366, 365, 346, 306, 198, 163, 145, 131, 105, 78, 39.

### 6. Stufe

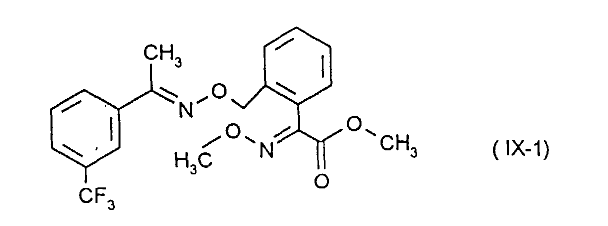

Die Herstellung dieser Verbindung ist in Beispiel 5 auf Seite 11 von EP 600 835 ausgehend von der Verbindung (X-1) beschrieben.

### (Variante 2)

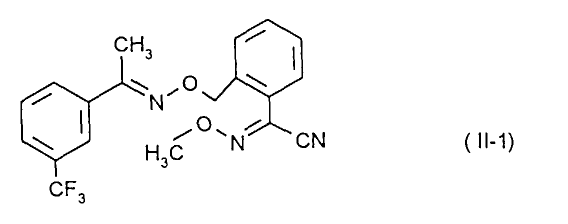

Zu 2,0 g (0,01 Mol) 3-Trifluormethylacetophenon-oxim in 25 ml trockenem Dimethylformamid werden zunächst portionsweise 0,3g (0,01 Mol) Natriumhydrid (80%-ig mit Mineralöl) unter kräftigem Rühren und danach 2,5 g (0,01 Mol) E-2-Methoximino-2-(2-brommethylphenyl)-acetonitril zugegeben.

Das Reaktionsgemisch wird 24 Stunden bei 60°C gerührt, anschließend auf Raumtemperatur abgekühlt, auf Wasser gegeben und mit Essigester ausgeschüttelt. Die organische Phase wird mit Wasser gewaschen, über Natriumsulfat getrocknet und im Vakuum eingeengt.

Man erhält 3,4 g (91% der Theorie) E-2-Methoximino-2-{2-[1-(3-trifluormethylphenyl)-ethylideniminoxymethyl]-phenyl}-acetonitril als gelbes Öl.

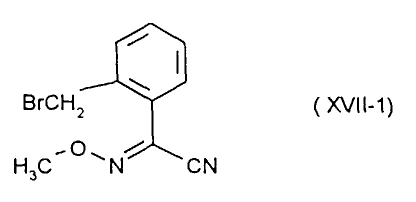

15,7 g (0,09 Mol) E-2-Methoximino-2-(2-methylphenyl)-acetonitril, 16,0 g (0,09 Mol) N-Bromsuccinimid und 0,45 g Dibenzoylperoxid werden in 130 ml Tetrachlormethan über Nacht unter Rückfluß zum Sieden erhitzt. Danach läßt man das Reaktionsgemisch auf Raumtemperatur abkühlen und filtriert. Die Mutterlauge wird im Vakuum eingeengt und der Rückstand über Kieselgel (Laufmittel: Hexan/Aceton 10 : 1) chromatographiert.

Man erhält 10,0 g (44% der Theorie) E-2-Methoximino-2-(2-brommethylphenyl)-acetonitril als hochviskoses Öl, das mittels Petrolether zur Kristallisation gebracht wird; Schmelzpunkt = 78-79°C.

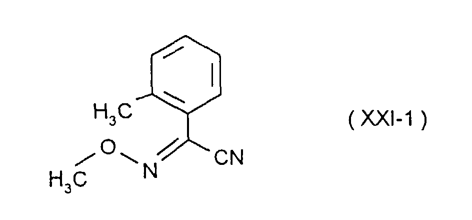

Zu einer Mischung von 77,2 g (0,4 Mol) E-2-Methoximino-2-(2-methylphenyl)-acetamid und 171,7 g Pyridin werden bei 0°C 138,6 g (0,66 Mol) Trifluoracetanhydrid getropft.

Man läßt 1 Stunde bei 0°C rühren, auf Raumtemperatur erwärmen und verrührt das Reaktionsgemisch anschließend mit 650 ml konzentrierter Salzsäure. Danach wird mit Essigester ausgeschüttelt, die organische Phase mit Wasser gewaschen, über Natriumsulfat getrocknet und im Vakuum eingeengt.

Man erhält 69,7 g (100% der Theorie) E-2-Methoximino-2-(2-methylphenyl)-acetonitril.
¹H-NMR (CDCl₃): d = 2,3 (s, 3 H).

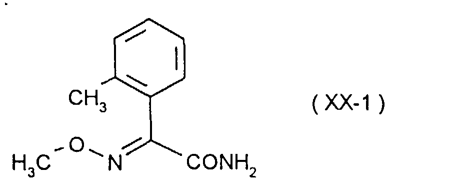

Zu 120 g (0,5 Mol) E-2-Methoximino-2-(2-methylphenyl)-essigsäuremethylester in 1,5 1 Methanol werden 1,5 1 25 %-iges Ammoniakwasser gegeben und 5 Stunden unter Rückfluß erhitzt. Danach läßt man das Reaktionsgemisch abkühlen, engt auf ca. 1 1 ein, verrührt mit Wasser und schüttelt mit Essigester aus. Die organische Phase wird mit Wasser gewaschen, über Natriumsulfat getrocknet und im Vakuum eingeengt. Der Rückstand wird mit Diisopropylether verrührt, abgesaugt und getrocknet.

Man erhält 66,2 g (69 % der Theorie) E-2-Methoximino-2-(2-methylphenyl)-acetamid vom Schmelzpunkt 98-99°C.

### Beispiel 2

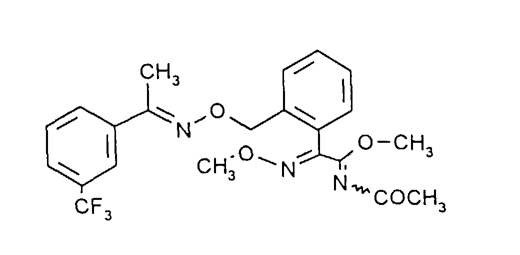

### (Verfahren b)

3,5 g (0 085 mol) E-2-Methoximino-2-{2-[1-(3-trifluormethylphenyl)-ethylideniminoxymethyl]-phenyl}-acetimidsäuremethylester (Beispiel 1) werden in 30 ml Pyridin gelöst, mit 0,8 g (0,0102 mol) Acetylchlorid versetzt und über Nacht bei Raumtemperatur gerührt. Das Lösungsmittel wird eingeengt, der Rückstand auf Wasser gegossen, mit Dichlormethan extrahiert, über Natriumsulfat getrocknet und wieder eingeengt. Man erhält 3,6 g eines gelben Öls mit einem Gehalt von 76 % (HPLC) N-Acetyl-2-(E-methoximino)-2-{2-[1-(3-trifluormethylphenyl)-ethylideniminoxymethyl]-phenyl}-acetimidsäuremethylester als Stereoisomerengemisch (Verhältnis ca. 1:7, nicht zugeordnet).
¹H-NMR (CDCl₃): d = 2,21; 2,26; 3,68: 3,97; 5,15: 7,15-7,95.

### Beispiel 3

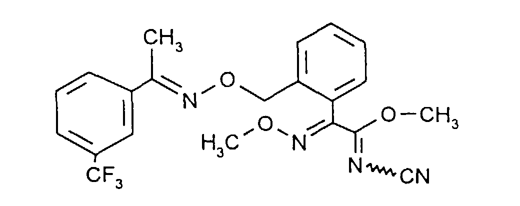

### (Verfahren c)

Zu 2,0 g (0,005 mol) E-2-Methoximino-2-{2-[1-(3-trifluormethylphenyl)-ethylideniminoxymethyl]-phenyl}-acetimidsäuremethylester (Beispiel 1) und 0,2 g (0,005 mol) Cyanamid gibt man eine Lösung von 3,6 g (0,01 mol) Dinatriumhydrogenphosphat und 1,6 g (0,01 mol) Natriumdihydrogenphosphat in 10 ml Wasser, sowie noch 10 ml Methanol und rührt über Nacht bei Raumtemperatur. Die Mischung wird mit Ether extrahiert, über Natriumsulfat getrocknet und eingeengt.

Der Rückstand wird mit Cyclohexan/Essigester (3:1) an Kieselgel chromatographiert. Man erhält 0,6 g (28 %) N-Cyan-2-(E-methoximino)-2-{2-[1-(3-trifluormethylphenyl)-ethyliden-iminoxymethyl]-phenyl}-acetimidsäuremethylestervom Schmelzpunkt 93°C.

### Beispiel 4

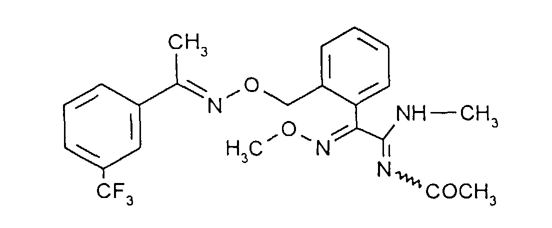

### (Verfahren d)

Zu einer Lösung von 1,1 g (0,0025 mol) N-Acetyl-2-(E-methoximino)-2-{2-[1-(3-trifluormethylphenyl)-ethyliden-iminoxymethyl]-phenyl}-acetimidsäuremethylester (Beispiel 2) in 30 ml Methanol wird unter Eiskühlung eine Lösung von 0,08 g (0,0025 mol) Methylamin in 30 ml Tetrahydrofuran gegeben. Es wird noch weitere 18 Stunden bei 20 °C gerührt. Die Lösung wird eingeengt und der Rückstand wird mit Cyclohexan/Essigester (3:1) an Kieselgel chromatographiert. Man erhält 0,44 g (39 % der Theorie) N-Acetyl-2-(E-methoximino)-2-{2-[1-(3-trifluormethylphenyl)-ethyliden-iminoxymethyl]-phenyl}-acetamidin.

### Beispiel 5

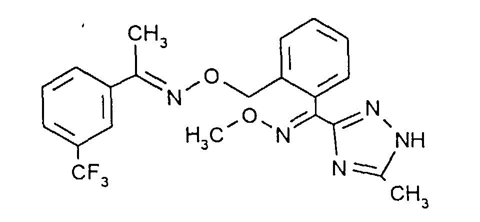

### (Verfahren e)

Zu einer Lösung von 1,1 g (0,0025 mol) N-Acetyl-2-(E-methoximino)-2-{2-[1-(3-trifluormethylphenyl)-ethyliden-iminoxymethyl]-phenyl}-acetimidsäuremethylester (Beispiel 2) in 30 ml Methanol wird unter Eiskühlung 0,08 g (0,0025 mol) Hydrazin gegeben. Es wird noch weitere 18 Stunden bei 20 °C gerührt. Die Lösung wird eingeengt und der Rückstand mit Cyclohexan/Essigester (3:1) an Kieselgel chromatographiert. Man erhält 0,42 g (39 % der Theorie) 5-Methyl-3-[E-methoximino-2-{2-[1-(3-trifluormethylphenyl)-ethyliden-iminoxymethyl]-phenyl}-triazol.

### Beispiel 6

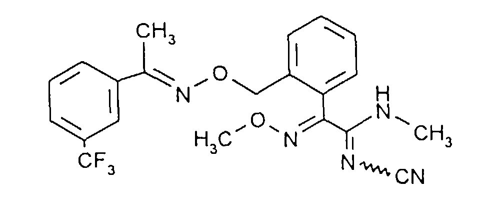

### (Verfahren d)

Zu einer Lösung von 1,4 g (0,0032 mol) N-Cyan-2-(E-methoximino)-2-{2-[1-(3-trifluormethylphenyl)-ethyliden-iminoxymethyl]-phenyl}-acetimidsäuremethylester (Beispiel 3) in 5 ml Methanol wird unter Eiskühlung 1g (0,032 mol) Methylamin eingeleitet. Es wird noch weitere 18 Stunden bei 20 °C gerührt. Die Lösung wird eingeengt und der Rückstand wird mit Cyclohexan/Essigester (3:1) an Kieselgel chromatographiert. Man erhält 0,42 g (31 % der Theorie) N-Cyan-N'-methyl-2-(E-methoximino)-2-{2-[1-(3-trifluormethylphenyl)-ethyliden-iminoxymethyl]-phenyl}-acetamidin.
¹H-NMR (CDCl₃): δ = 2,25; 4,05; 5,11 ppm.

### Beispiel 7

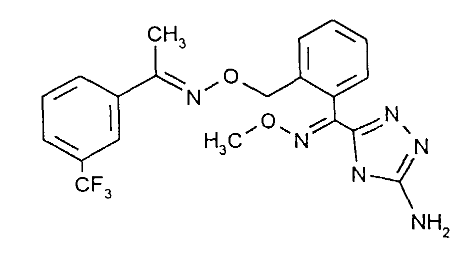

### (Verfahren e)

Zu einer Lösung von 1,4 g (0,0032 mol) N-Cyan-2-(E-methoximino)-2-{2-[1-(3-trifluormethylphenyl)-ethyliden-iminoxymethyl]-phenyl}-acetimidsäuremethylester (Beispiel 3) in 10 ml Methanol wird unter Eiskühlung 0,1 g (0,0032 mol) Hydrazin gegeben. Es wird noch weitere 18 Stunden bei 20°C gerührt. Die Lösung wird eingeengt und der Rückstand mit Cyclohexan/Essigester (3:1) an Kieselgel chromatographiert. Man erhält 0,46 g (33 % der Theorie) 5-Amino-3-[E-methoximino-2-{2-[1-(3-trifluormethylphenyl)-ethyliden-iminoxymethyl]-phenyl}-triazol.

### Beispiel 8

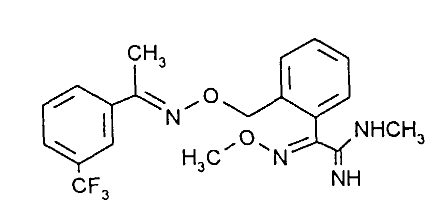

### (Verfahren f)

2,4 g (0,006 mol) E-2-Methoximino-2-{2-[1-(3-trifluormethylphenyl)-ethylideniminoxymethyl]-phenyl}-acetimidsäuremethylester (Beispiel 1) werden in 40 ml 30%-iger Methylamin-Lösung in Ethanol 24 Stunden unter Rückfluß zum Sieden erhitzt. Danach wird die Reaktionsmischung im Vakuum eingeengt. Man erhält 2,5 g (100 % der Theorie) E-2-Methoximino)-2-{2-[1-(3-trifluormethylphenyl)-ethylideniminoxymethyl]-phenyl}-acetimidsäuremethylamid als Öl.
¹H-NMR (CDCl₃): d = 2,95 (s, 3H).

Analog den Herstellungsbeispielen 1 bis 8 sowie entsprechend der allgemeinen Beschreibung der erfindungsgemäßen Verfahren werden die in der nachstehenden Tabelle aufgeführten Verbindungen der allgemeinen Formel (I) erhalten:

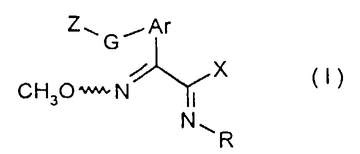

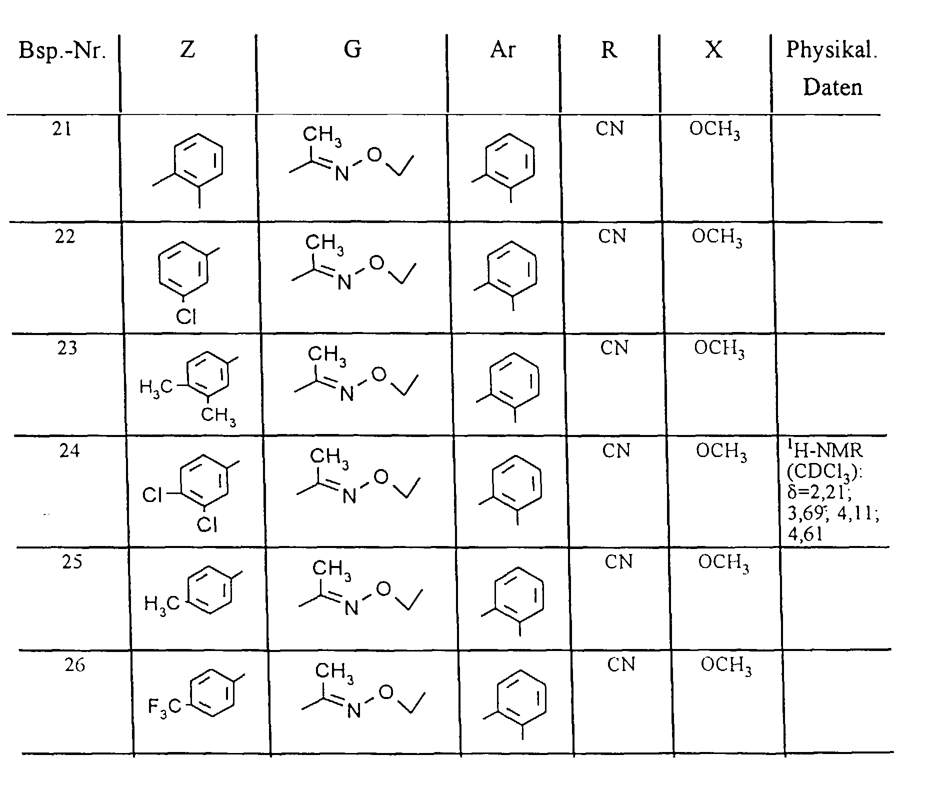

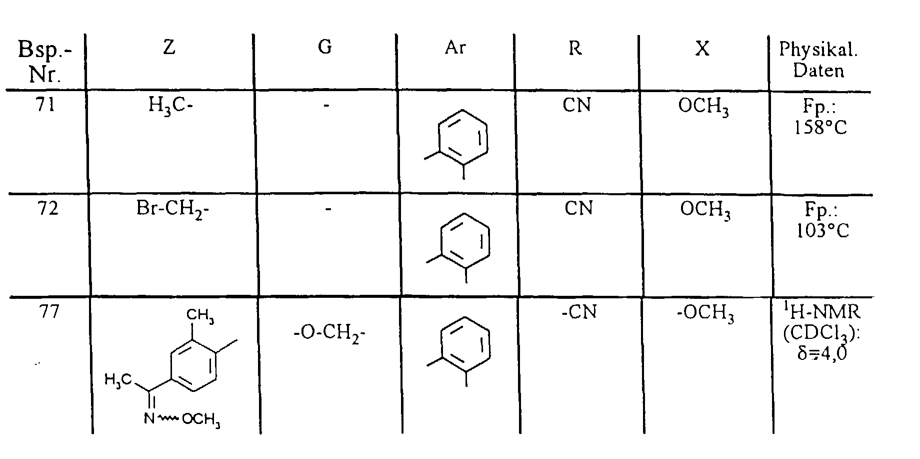

### Anwendungsbeispiele

### Beispiel A

### Podosphaera-Test (Apfel) / protectiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen durch Bestäuben mit Konidien des Apfelmehltauerregers Podosphaera leucotricha inokuliert.

Die Pflanzen werden dann im Gewächshaus bei 23°C und einer relativen Luftfeuchtigkeit von ca. 70% aufgestellt.

10 Tage nach der Inokulation erfolgt die Auswertung.

In diesem Test zeigt die Verbindung gemäß Herstellungsbeispiel 1 bei einer Wirkstoffkonzentration von 20 ppm einen Wirkungsgrad von bis zu 100 %.

### Beispiel B

### Venturia-Test (Apfel) / protectiv

| | |
|---|---|
| Lösungsmittel | 4,7 Gewichtsteile Aceton |
| Emulgator | 0,3 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit einer wäßrigen Konidiensuspension des Apfelschorferregers Venturia inaequalis inokuliert und verbleiben dann 1 Tag bei 20°C und 100% relativer Luftfeuchtigkeit in einer Inkubationskabine.

Die Pflanzen werden dann im Gewächshaus bei 20°C und einer relativen Luftfeuchtigkeit von ca. 70% aufgestellt.

12 Tage nach der Inokulation erfolgt die Auswertung.

In diesem Test zeigt die Verbindung gemäß Herstellungsbeispiel 1 einen ausgezeichneten Wirkungsgrad.

### Beispiel C

### Erysiphe-Test (Gerste) / protectiv

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp. hordei bestäubt.

Die Pflanzen werden dann im Gewächshaus bei einer Temperatur von ca. 20°C und einer relativen Luftfeuchtigkeit von ca. 80% aufgestellt, um die Entwicklung von Methltaupusteln zu begünstigen.

7 Tage nach der Inokulation erfolgt die Auswertung.

In diesem Test zeigt die Verbindung gemäß Herstellungsbeispiel 1 bei einer Wirkstoffmenge von 250g/ha einen Wirkungsgrad von 100%.

### Beispiel D

### Erysiphe-Test (Gerste) / kurativ

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden dann im Gewächshaus bei einer Temperatur von ca. 20°C und einer relativen Luftfeuchtigkeit von ca. 80% aufgestellt, um die Entwicklung von Methltaupusteln zu begünstigen.

7 Tage nach der Inokulation erfolgt die Auswertung.

In diesem Test zeigt die Verbindung gemäß Herstellungsbeispiel 1 bei einer Wirkstoffmenge von 250g/ha einen Wirkungsgrad von 100%.

### Beispiel E

### Erysiphe-Test (Weizen) / protectiv

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung in der angegebenen Aufwandmenge. Nach Antrocknen des Spritzbelages werden die Pflanzen mit Sporen von Erysiphe graminis f.sp. hordei bestäubt.

Die Pflanzen werden dann im Gewächshaus bei einer Temperatur von ca. 20°C und einer relativen Luftfeuchtigkeit von ca. 80% aufgestellt, um die Entwicklung von Methltaupusteln zu begünstigen.

7 Tage nach der Inokulation erfolgt die Auswertung.

In diesem Test zeigt die Verbindung gemäß Herstellungsbeispiel 1 bei einer Wirkstoffmenge von 250g/ha einen Wirkungsgrad von 100%.

### Beispiel F

### Erysiphe-Test (Weizen) / kurativ

| | |
|---|---|
| Lösungsmittel | 10 Gewichtsteile N-Methyl-pyrrolidon |
| Emulgator | 0,6 Gewichtsteile Alkyl-Aryl-Polyglykolether |

Zur Herstellung einer zweckmäßigen Wirkstoffzubereitung vermischt man 1 Gewichststeil Wirkstoff mit den angegebenen Mengen Lösungsmittel und Emulgator und verdünnt das Konzentrat mit Wasser auf die gewünschte Konzentration.

Zur Prüfung auf kurative Wirksamkeit werden junge Pflanzen mit Sporen von Erysiphe graminis f.sp. hordei bestäubt. 48 Stunden nach der Inokulation werden die Pflanzen mit der Wirkstoffzubereitung in der angegebenen Aufwandmenge besprüht.

Die Pflanzen werden dann im Gewächshaus bei einer Temperatur von ca. 20°C und einer relativen Luftfeuchtigkeit von ca. 80% aufgestellt, um die Entwicklung von Methltaupusteln zu begünstigen.

7 Tage nach der Inokulation erfolgt die Auswertung.

In diesem Test zeigt die Verbindung gemäß Herstellungsbeispiel 1 bei einer Wirkstoffmenge von 250g/ha einen Wirkungsgrad von 100%.

## Patentansprüche

1. Imidsäurederivate der allgemeinen Formel (I), 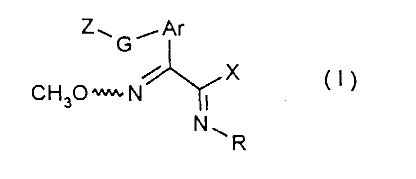 in welcher
R für Cyano steht;
X für Alkoxy, Alkylthio, Amino, Alkylamino oder Dialkylamino steht, oder
X und R gemeinsam mit dem Kohlenstoff- bzw. Stickstoff-Atom, an das sie gebunden sind, für einen Triazolrest der Formel 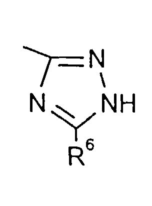 stehen, wobei
R⁶ für Alkyl, Hydroxy, Mercapto, Amino, Alkylamino oder Dialkylamino steht;
Ar für jeweils gegebenenfalls substituiertes Arylen oder Heteroarylen steht;
G für eine Einfachbindung, für Sauerstoff, für jeweils gegebenenfalls durch Halogen, Hydroxy, Alkyl, Halogenalkyl oder Cycloalkyl substituiertes Alkandiyl, Alkendiyl, Oxaalkendiyl, Alkindiyl oder eine der nachstehenden Gruppierungen steht:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-O-,
wobei
Q für Sauerstoff oder Schwefel steht,
n für die Zahlen 0, 1 oder 2 steht,
R⁴ für Wasserstoff, Cyano oder jeweils gegebenenfalls substituiertes Alkyl, Alkoxy, Alkylthio, Alkylamino, Dialkylamino oder Cycloalkyl steht, und
R⁵ für Wasserstoff, Hydroxy, Cyano oder jeweils gegebenenfalls substituiertes Alkyl, Alkoxy oder Cycloalkyl steht; und
Z für jeweils gegebenenfalls substituiertes Alkyl, Alkenyl, Alkinyl, Cycloalkyl, Aryl oder Heterocyclyl steht;
wobei die Verbindungen ausgenommen sind, in denen gleichzeitig R für Wasserstoff steht, G für Sauerstoff oder die Gruppierung -OCH₂- steht und Z für jeweils gegebenenfalls substituiertes Aryl oder 2-Pyridyl steht.

2. Verbindungen der Formel (I) gemäß Anspruch 1, in welcher
R für Cyano steht;
X für jeweils geradkettiges ofer verzweigtes Alkoxy oder Alkylthio mit jeweils 1 bis 4 Kohlenstoffatomen;
Amino oder jeweils geradkettiges oder verzweigtes Alkylamino und Dialkylamino mit jeweils 1 bis 4 Kohlenstoffatomen in den einzelnen Alkylteilen steht.
X und R gemeinsam mit dem Kohlenstoff- bzw. Stickstoff-Atom, an das sie gebunden sind,
für einen Triazolrest der Formel 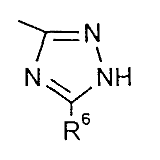 stehen, wobei
R⁶ für geradkettiges oder verzweigtes Alkyl mit 1 bis 4 Kohlenstoffatomen, Hydroxy, Mercapto, Amino oder jeweils geradkettiges oder verzweigtes Alkylamino und Dialkylamino mit jeweils 1 bis 4 Kohlenstoffatomen in den einzelnen Alkylteilen steht.
Ar für jeweils gegebenenfalls substituiertes Phenylen oder Naphthylen oder für Heteroarylen mit 5 oder 6 Ringgliedern, von denen mindestens eines für Sauerstoff, Schwefel oder Stickstoff steht und gegebenenfalls ein oder zwei weitere für Stickstoff stehen, steht, wobei die möglichen Substituenten aus der nachstehenden Aufzählung ausgewählt sind:
Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl, Alkenyloxy oder Alkinyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen.
G für eine Einfachbindung, für Sauerstoff, für jeweils gegebenenfalls durch Halogen, Hydroxy, C₁-C₄-Alkyl, C₁-C₄-Halogenalkyl oder C₃-C₆-Cycloalkyl substituiertes Alkandiyl, Alkendiyl, Oxaalkendiyl, Alkindiyl mit jeweils bis zu 4 Kohlenstoffatomen oder eine der nachstehenden Gruppierungen steht:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- oder -N=N-C(R⁴)=N-,
wobei
n für die Zahlen 0, 1 oder 2 steht,
Q für Sauerstoff oder Schwefel steht,
R⁴ für Wasserstoff, Cyano, für gegebenenfalls durch Halogen, Cyano oder C₁-C₄-Alkoxy substituiertes Alkyl, Alkoxy, Alkylthio, Alkylamino oder Dialkylamino mit jeweils 1 bis 6 Kohlenstoffatomen in den Alkylgruppen oder für jeweils gegebenenfalls durch Halogen, Cyano, Carboxy, C₁-C₄-Alkyl oder C₁-C₄-Alkoxy-carbonyl substituiertes Cycloalkyl mit 3 bis 6 Kohlenstoffatomen steht, und
R⁵ für Wasserstoff, Hydroxy, Cyano oder für gegebenenfalls durch Halogen, Cyano oder C₁-C₄-Alkoxy substituiertes Alkyl mit 1 bis 6 Kohlenstoffatomen oder für gegebenenfalls durch Halogen, Cyano, Carboxy, C₁-C₄-Alkyl oder C1-C4-Alkoxy-carbonyl substituiertes Cycloalkyl mit 3 bis 6 Kohlenstoffatomen steht.
Z für gegebenenfalls durch Halogen, Cyano, Hydroxy, Amino, C₁-C₄-Alkoxy, C₁-C₄-Alkylthio, C₁-C₄-Alkylsulfinyl oder C₁-C₄-Alkylsulfonyl (welche jeweils gegebenenfalls durch Halogen substituiert sind) substituiertes Alkyl mit 1 bis 8 Kohlenstoffatomen, für jeweils gegebenenfalls durch Halogen substituiertes Alkenyl oder Alkinyl mit jeweils bis zu 8 Kohlenstoffatomen, für jeweils gegebenenfalls durch Halogen, Cyano, Carboxy, Phenyl (welches gegebenenfalls durch Halogen, Cyano, C₁-C₄-Alkyl, C₁-C₄-Halogenalkyl, C₁-C₄-Alkoxy oder C₁-C₄-Halogenalkoxy substituiert ist), C₁-C₄-Alkyl oder C₁-C₄-Alkoxy-carbonyl substiuiertes Cycloalkyl mit 3 bis 6 Kohlenstoffatomen, für jeweils gegebenenfalls substituiertes Phenyl, Naphthyl oder (gegebenenfalls benzannelliertes) Heterocyclyl mit 5 oder 6 Ringgliedern, von denen mindestens eines für Sauerstoff, Schwefel oder Stickstoff und gegebenenfalls ein oder zwei weitere für Stickstoff stehen, steht, wobei die möglichen Substituenten aus der nachstehenden Aufzählung ausgewählt sind:
Sauerstoff (als Ersatz für zwei geminale Wasserstoffatome), Halogen, Cyano, Nitro, Amino, Hydroxy, Formyl, Carboxy, Carbamoyl, Thiocarbamoyl, jeweils geradkettiges oder verzweigtes Alkyl, Alkoxy, Alkylthio, Alkylsulfinyl oder Alkylsulfonyl mit jeweils 1 bis 6 Kohlenstoffatomen, jeweils geradkettiges oder verzweigtes Alkenyl oder Alkenyloxy mit jeweils 2 bis 6 Kohlenstoffatomen, jeweils geradkettiges oder verzweigtes Halogenalkyl, Halogenalkoxy, Halogenalkylthio, Halogenalkylsulfinyl oder Halogenalkylsulfonyl mit jeweils 1 bis 6 Kohlenstoffatomen und 1 bis 13 gleichen oder verschiedenen Halogenatomen, jeweils geradkettiges oder verzweigtes Halogenalkenyl oder Halogenalkenyloxy mit jeweils 2 bis 6 Kohlenstoffatomen und 1 bis 13 gleichen oder verschiedenen Halogenatomen, jeweils geradkettiges oder verzweigtes Alkylamino, Dialkylamino, Alkylcarbonyl, Alkylcarbonyloxy, Alkoxycarbonyl, Alkylsulfonyloxy, Hydroximinoalkyl oder Alkoximinoalkyl mit jeweils 1 bis 6 Kohlenstoffatomen in den einzelnen Alkylteilen, jeweils gegebenenfalls einfach oder mehrfach, gleich oder verschieden durch Halogen und/oder geradkettiges oder verzweigtes Alkyl mit 1 bis 4 Kohlenstoffatomen und/oder geradkettiges oder verzweigtes Halogenalkyl mit 1 bis 4 Kohlenstoffatomen und 1 bis 9 gleichen oder verschiedenen Halogenatomen substituiertes, jeweils zweifach verknüpftes Alkylen oder Dioxyalkylen mit jeweils 1 bis 6 Kohlenstoffatomen, Cycloalkyl mit 3 bis 6 Kohlenstoffatomen, Heterocyclyl oder Heterocyclyl-methyl mit jeweils 3 bis 7 Ringgliedern, von denen jeweils 1 bis 3 gleiche oder verschiedene Heteroatome sind - insbesondere Stickstoff, Sauerstoff und/oder Schwefel -, sowie jeweils gegebenenfalls im Phenylteil einfach oder mehrfach, gleich oder verschieden durch Halogen, Cyano, Nitro, Carboxy, Carbamoyl und/oder geradkettiges oder verzweigtes Alkyl mit 1 bis 4 Kohlenstoffatomen und/oder geradkettiges oder verzweigtes Halogenalkyl mit 1 bis 4 Kohlen. stoffatomen und 1 bis 9 gleichen oder verschiedenen Halogenatomen und/oder geradkettiges oder verzweigtes Alkoxy mit 1 bis 4 Kohlenstoffatomen und/oder geradkettiges oder verzweigtes Halogenalkoxy mit 1 bis 4 Kohlenstoffatomen und 1 bis 9 gleichen oder verschiedenen Halogenatomen und/oder Alkylcarbonyl oder Alkoxycarbonyl mit jeweils bis zu 5 Kohlenstoffatomen substituiertes Phenyl, Phenoxy, Benzyl, Benzyloxy, Phenylethyl oder Phenylethyloxy.

3. Verbindungen der Formel (I)gemäß Anspruch 1, in welcher
R für Cyano steht;
X für Methoxy, Ethoxy, n- oder i-Propoxy, n-, i-, s- oder t-Butoxy; Methylthio, Ethylthio, n- oder i-Propylthio, n-, i-, s- oder t-Butylthio; Amino, Methylamino, Ethylamino, n- oder i-Propylamino; Dimethylamino, Methyl-ethyl-amino, Diethylamino, Methyl-n- oder i-Propylamino und Ethyl-n- oder i-Propylamino steht.
X und R gemeinsam mit dem Kohlenstoff- bzw. Stickstoff-Atom, an das sie gebunden sind, für einen Triazolrest der Formel 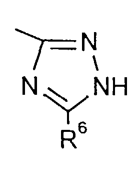 stehen, wobei
R⁶ für Methyl, Ethyl, n- oder i-Propyl, n-, i-, s- oder t-Butyl; Hydroxy; Mercapto; Amino, Methylamino, Ethylamino, n- oder i-Propylamino; Dimethylamino, Methyl-ethyl-amino, Diethylamino, Methyl-n- oder i-Propylamino und Ethyl-n- oder i-Propylamino steht.
Ar für jeweils gegebenenfalls substituiertes ortho-, meta- oder para-Phenylen, für Furandiyl, Thiophendiyl, Pyrroldiyl, Pyrazoldiyl, Triazoldiyl, Oxazoldiyl, Isoxazoldiyl, Thiazoldiyl, Isothiazoldiyl, Oxadiazoldiyl, Thiadiazoldiyl, Pyridindiyl, Pyrimidindiyl, Pyridazindiyl, Pyrazindiyl, 1,3,4-Triazindiyl oder 1,2,3-Triazindiyl steht, wobei die möglichen Substituenten insbesondere aus der nachstehenden Aufzählung ausgewählt sind:
Fluor, Chlor, Cyano, Methyl, Ethyl, Trifluormethyl, Methoxy, Ethoxy, Methylthio, Methylsulfinyl oder Methylsulfonyl.
G für eine Einfachbindung, für Sauerstoff, für jeweils gegebenenfalls durch Fluor, Chlor, Hydroxy, Methyl, Ethyl, n- oder i-Propyl, Trifluormethyl, Cyclopropyl, Cyclobutyl, Cyclopentyl oder Cyclohexyl substituiertes Methylen, Dimethylen (Ethan-1,2-diyl), Ethen-1,2-diyl, Ethin-1,2-diyl oder eine der nachstehenden Gruppierungen
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, - S(O)ₙ-CH₂,-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- oder -N(R⁵)-CQ-Q-CH₂- steht,
wobei
n für die Zahlen 0, 1 oder 2 steht,
Q für Sauerstoff oder Schwefel steht,
R⁴ für Wasserstoff, Cyano, für gegebenenfalls durch Fluor, Chlor, Cyano, Methoxy oder Ethoxy substituiertes Methyl, Ethyl, n- oder i-Propyl, n-, i- oder s-Butyl, Methoxy, Ethoxy, Propoxy, Butoxy, Methylthio, Ethylthio, Propylthio, Butylthio, Methylamino, Ethylamino, Propylamino, Dimethylamino oder Diethylamino oder für jeweils gegebenenfalls durch Fluor, Chlor, Cyano, Carboxy, Methyl, Ethyl, n- oder i-Propyl, Methoxycarbonyl oder Ethoxy-carbonyl substituiertes Cyclopropyl, Cyclobutyl, Cyclopentyl oder Cyclohexyl steht, und
R⁵ für Wasserstoff, Hydroxy, Cyano oder für jeweils gegebenenfalls durch Fluor, Chlor, Cyano, Methoxy oder Ethoxy substituiertes Methyl, Ethyl, n- oder i-Propyl, n-, i-, s- oder t-Butyl oder für gegebenenfalls durch Fluor, Chlor, Cyano, Carboxy, Methyl, Ethyl, n- oder i-Propyl, Methoxy-carbonyl oder Ethoxy-carbonyl substituiertes Cyclopropyl, Cyclobutyl, Cyclopentyl oder Cyclohexyl steht.
Z für gegebenenfalls durch Fluor, Chlor, Brom, Cyano, Hydroxy, Amino, Methoxy, Ethoxy, Methylthio, Ethylthio, Methylsulfinyl, Ethylsulfinyl, Methylsulfonyl, Ethylsulfonyl (welche jeweils gegebenenfalls durch Fluor und/oder Chlor substituiert sind) substituiertes Methyl, Ethyl, n- oder i-Propyl, n-, i-, s- oder t-Butyl, für jeweils gegebenenfalls durch Fluor, Chlor oder Brom substituiertes Allyl, Crotonyl, 1-Methyl-allyl, Propargyl oder 1-Methyl-propargyl, für jeweils gegebenenfalls durch Fluor, Chlor, Brom, Cyano, Carboxy, Phenyl (welches gegebenenfalls durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiert ist), Methyl, Ethyl, n- oder i-Propyl, Methoxy-carbonyl oder Ethoxy-carbonyl substiuiertes Cyclopropyl, Cyclobutyl, Cyclopentyl oder Cyclohexyl, für jeweils gegebenenfalls substituiertes Phenyl, Naphthyl, Furyl, Tetrahydrofuryl, Benzofuryl, Tetrahydropyranyl, Thienyl, Benzothienyl, Pyrrolyl, Dihydropyrrolyl, Tetrahydropyrrolyl, Benzopyrrolyl, Benzodihydropyrrolyl, Oxazolyl, Benzoxazolyl, Isoxazolyl, Thiazolyl, Benzthiazolyl, Isothiazolyl, Imidazolyl, Benzimidazolyl, Oxadiazolyl, Thiadiazolyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Sauerstoff (als Ersatz für zwei geminale Wasserstoffatome), Fluor, Chlor, Brom, Cyano, Nitro, Amino, Hydroxy, Formyl, Carboxy, Carbamoyl, Thiocarbamoyl, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methylamino, Ethyl. amino, n- oder i-Propylamino, Dimethylamino, Diethylamino, Acetyl, Propionyl, Acetyloxy, Methoxycarbonyl, Ethoxycarbonyl, Methylsulfonyloxy, Ethylsulfonyloxy, Hydroximinomethyl, Hydroximinoethyl, Methoximinomethyl, Ethoximinomethyl, Methoximinoethyl oder Ethoximinoethyl; jeweils gegebenenfalls einfach oder mehrfach, gleich oder verschieden durch Fluor, Chlor, Methyl, Ethyl, n- oder i-Propyl substituiertes Trimethylen (Propan-1,3-diyl), Methylendioxy oder Ethylendioxy, Cyclopropyl, Cyclobutyl, Cyclopentyl oder Cyclohexyl, sowie jeweils gegebenenfalls im Phenylteil einfach oder mehrfach, gleich oder verschieden durch Fluor, Chlor, Brom, Cyano, Nitro, Carboxy, Carbamoyl, Methyl, Ethyl, n- oder i-Propyl, n-, i-, s- oder t-Butyl, Trifluormethyl, Methoxy, Ethoxy, n- oder i-Propoxy, Difluormethoxy, Trifluormethoxy, Acetyl, Methoxy-carbonyl oder Ethoxycarbonyl substituiertes Phenyl, Phenoxy, Benzyl oder Benzyloxy.

4. Verbindungen der Formel (I) gemäß Anspruch 1, in welcher
R Cyano steht;
X für Methoxy, Ethoxy, Methylthio, Ethylthio, Amino, Methylamino, Ethylamino, Dimethylamino oder Diethylamino steht.
X und R gemeinsam mit dem Kohlenstoff- bzw. Stickstoff-Atom, an das sie gebunden sind, für einen Triazolrest der Formel 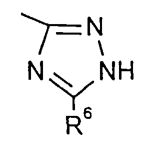 stehen, wobei
R⁶ für Methyl, Ethyl, Hydroxy, Mercapto oder Amino steht.
Ar für ortho-Phenylen, Pyridin-2,3-diyl oder Thiophen-2,3-diyl steht.
G für Sauerstoff, Methylen oder eine der nachstehenden Gruppierungen
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -O-C(R⁴)=N-O-CH₂-, -NH-C(R⁴)=N-O-CH₂-, -N(R⁵)- oder -CH₂-O-N=C(R⁴)-steht,
wobei
n für die Zahlen 0, 1 oder 2 steht,
R⁴ für Wasserstoff, Methyl oder Ethyl steht und
R⁵ für Wasserstoff, Methyl oder Ethyl steht.
Z für jeweils gegebenenfalls substituiertes Phenyl, Pyridinyl, Pyrimidinyl, Pyridazinyl, Pyrazinyl, 1,2,3-Triazinyl, 1,2,4-Triazinyl oder 1,3,5-Triazinyl steht, wobei die möglichen Substituenten aus der nachstehenden Aufzählung ausgewählt sind:
Fluor, Chlor, Brom, Cyano, Methyl, Ethyl, n- oder i-Propyl, n-, i-, s- oder t-Butyl, Methoxy, Ethoxy, n- oder i-Propoxy, Methylthio, Ethylthio, n- oder i-Propylthio, Methylsulfinyl, Ethylsulfinyl, Methylsulfonyl oder Ethylsulfonyl, Trifluormethyl, Difluormethoxy, Trifluormethoxy, Difluormethylthio, Trifluormethylthio, Trifluormethylsulfinyl oder Trifluormethylsulfonyl, Methoxycarbonyl, Ethoxycarbonyl, Methoximinomethyl, Ethoximinomethyl, Methoximinoethyl, Ethoximinoethyl, jeweils gegebenenfalls einfach oder mehrfach, gleich oder verschieden durch Fluor, Chlor, Methyl oder Ethyl substituiertes Methylendioxy oder Ethylendioxy, sowie jeweils gegebenenfalls im Phenylteil einfach oder mehrfach, gleich oder verschieden durch Fluor, Chlor, Brom, Cyano, Methyl, Ethyl, n- oder i-Propyl, n-, i-, s- oder t-Butyl, Trifluormethyl, Methoxy, Ethoxy, n- oder i-Propoxy, Difluormethoxy oder Trifluormethoxy substituiertes Phenyl, Phenoxy, Benzyl oder Benzyloxy.

5. Verbindungen der Formel (I) gemäß Anspruch 1, in welcher
Ar für ortho-Phenylen steht.

6. Schädlingsbekämpfungsmittel, gekennzeichnet durch einen Gehalt an mindestens einer Verbindung der Formel (I) nach Anspruch 1.

7. Verfahren zur Bekämpfung von Schädlingen, dadurch gekennzeichnet, daß man Verbindungen der Formel (I) nach Anspruch 1 auf Schädlinge und/oder ihren Lebensraum einwirken läßt.

8. Verfahren zur Herstellung von Verbindungen der Formel (I) nach Anspruch 1, dadurch gekennzeichnet, daß man
a) Nitrile der allgemeinen Formel (II) 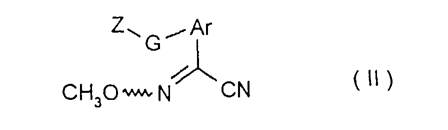 in welcher
Ar, G und Z die in Anspruch 1 angegebene Bedeutung haben,
mit (Thio) Alkoholen der allgemeinen Formel (III)
H-X¹ (III)
in welcher
X¹ für Alkoxy oder Alkylthio steht,
bzw. deren Alkalimetallsalzen, gegebenenfalls in Gegenwart eines Verdünnungsmittels und gegebenenfalls in Gegenwart eines Reaktionshilfsmittels umsetzt;
oder
b) die gemäß Verfahren (a) erhältlichen Imidoester der allgemeinen Formel (Ia) 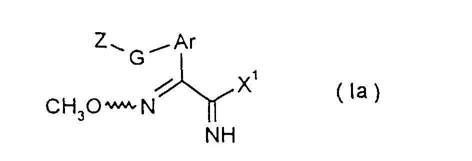 in welcher
Ar, G, Z und X¹ die oben angegebene Bedeutung haben,
mit reaktiven Säurederivaten, insbesondere mit Säurehalogeniden der allgemeinen Formel (IV)
Hal-R (IV)
in welcher
R die in Anspruch 1 angegebene Bedeutung hat und
Hal für Halogen steht,
bzw. den entsprechenden Anhydriden, gegebenenfalls in Gegenwart eines Verdünnungsmittels und gegebenenfalls in Gegenwart eines Reaktionshilfsmittels umsetzt;
oder
c) die gemäß Verfahren (a) erhältlichen Imidoester der allgemeinen Formel (Ia) 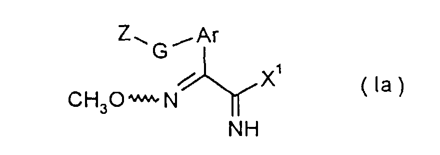 in welcher
Ar, G, Z und X¹ die oben angegebene Bedeutung haben,
mit Cyanamid der Formel (V)
NH₂-CN (V)
gegebenenfalls in Gegenwart eines Verdünnungsmittels und gegebenenfalls in Gegenwart eines Reaktionshilfsmittels umsetzt;
oder
d) die gemäß den Verfahren (b) und (c) erhältlichen Imidsäurederivate der allgemeinen Formel (Ib) 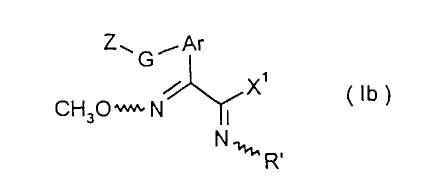 in welcher
Ar, G, Z und X¹ die oben angegebene Bedeutung haben und
R' für Cyano steht,
mit Aminen der allgemeinen Formel (VI)
H-X² (VI)
in welcher
X² für Amino, Alkylamino oder Dialkylamino steht,
gegebenenfalls in Gegenwart eines Verdünnungsmittels umsetzt;
oder
e) die gemäß den Verfahren (b) und (c) erhältlichen Imidsäurederivate der allgemeinen Formel (Ic) 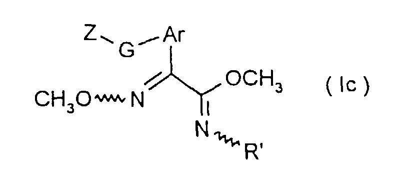 in welcher
Ar, G, Z und R' die oben angegebene Bedeutung haben,
mit Hydrazin bzw. Hydrazinhydrat, gegebenenfalls in Gegenwart eines Verdünnungsmittels umsetzt;
oder
f) die gemäß Verfahren (a) erhältlichen Imidoester der allgemeinen Formel (Ia) 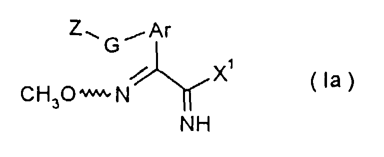 in welcher
Ar, G, Z und X¹ die oben angegebene Bedeutung haben,
mit Aminen der allgemeinen Formel (VI)
H-X² (VI)
in welcher
X² die oben angegebene Bedeutung hat,
gegebenenfalls in Gegenwart eines Verdünnungsmittels umsetzt.

9. Verwendung von Verbindungen der Formel (I) nach den Ansprüchen 1 bis 5 zur Bekämpfung von Schädlingen.

10. Verfahren zur Herstellung von Schädlingsbekämpfungsmitteln, dadurch gekennzeichnet, daß man Verbindungen der Formel (I) nach den Ansprüchen 1 bis 5 mit Streckmitteln und/oder oberflächenaktiven Mitteln vermischt.

11. Verbindungen Verbindungen der Formel (Ia) 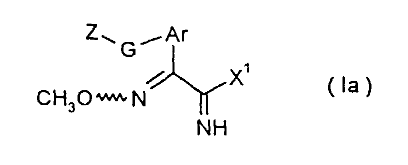 in welcher
Ar, G und Z die in Anspruch 1 angegebene Bedeutung haben und
X¹ für Alkoxy oder Alkylthio steht.

12. Verbindungen der Formel (Ib) 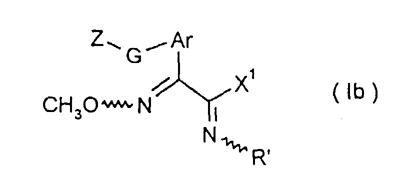 in welcher
Ar, G, Z und X¹ die in Anspruch 11 angegebene Bedeutung haben und
R' für Cyano steht.

13. Verbindungen der Formel (Ic) 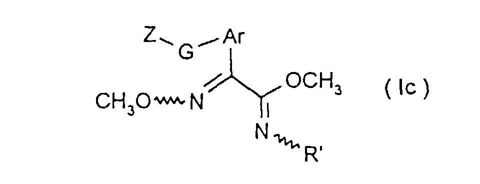 in welcher
Ar, G, Z und R' die in Anspruch 12 angegebene Bedeutung haben.

## Claims

1. Imidic acid derivatives of the general formula (I) 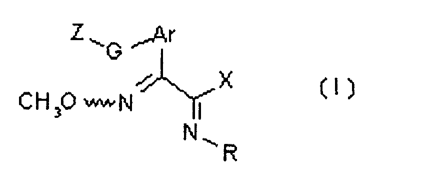 in which
R represents cyano;
X represents alkoxy, alkylthio, amino, alkylamino or dialkylamino, or
X and R together with the carbon or nitrogen atom to which they are bonded
represent a triazole radical of the formula 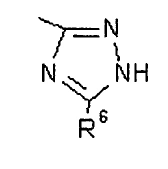 where
R⁶ represents alkyl, hydroxyl, mercapto, amino, alkylamino or dialkylamino;
Ar represents in each case optionally substituted arylene or heteroarylene;
G represents a single bond, represents oxygen, or represents in each case optionally halogen-, hydroxyl-, alkyl-, halogenoalkyl- or cycloalkyl-substituted alkanediyl, alkenediyl, oxaalkenediyl, alkinediyl or one of the following groups:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- or -N=N-C (R⁴)=N-O-,
where
Q represents oxygen or sulphur,
n represents the numbers 0, 1 or 2,
R⁴ represents hydrogen, cyano or in each case optionally substituted alkyl, alkoxy, alkylthio, alkylamino, dialkylamino or cycloalkyl, and
R⁵ represents hydrogen, hydroxyl, cyano or in each case optionally substituted alkyl, alkoxy or cycloalkyl; and
Z represents in each case optionally substituted alkyl, alkenyl, alkinyl, cycloalkyl, aryl or heterocyclyl,
with the exception of those compounds in which simultaneously R represents hydrogen, G represents oxygen or the group -OCH₂- and Z represents in each case optionally substituted aryl or 2-pyridyl.

2. Compounds of the formula (I) according to Claim 1, in which
R represents cyano;
X represents in each case straight-chain or branched alkoxy or alkylthio, each of which has 1 to 4 carbon atoms;
amino or in each case straight-chain or branched alkylamino and dialkylamino, each of which has 1 to 4 carbon atoms in the individual alkyl moieties,
X and R together with the carbon or nitrogen atom to which they are bonded represent a triazole
radical of the formula 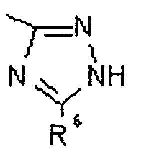 where
R⁶ represents straight-chain or branched alkyl having 1 to 4 carbon atoms, hydroxyl, mercapto, amino or in each case straight-chain or branched alkylamino and dialkylamino, each of which has 1 to 4 carbon atoms in the individual alkyl moieties,
Ar represents in each case optionally substituted phenylene or naphthylene, or represents heteroarylene having 5 or 6 ring members of which at least one represents oxygen, sulphur or nitrogen and, if appropriate, one or two further ring members represent nitrogen, the substituents which are possible being selected from the following enumeration:
halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl, alkenyloxy or alkinyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, and in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms,
G represents a single bond, represents oxygen, or represents in each case optionally halogen-, hydroxyl-, C₁-C₄-alkyl-, C₁-C₄-halogenoalkyl- or C₃-C₆-cycloalkyl-substituted alkanediyl, alkenediyl, oxaalkenediyl or alkinediyl, each of which has up to 4 carbon atoms, or one of the groups which follow:
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- or -N=N-C(R⁴)=N-,
where
n represents the numbers 0, 1 or 2,
Q represents oxygen or sulphur,
R⁴ represents hydrogen, cyano, or represents alkyl, alkoxy, alkylthio, alkylamino or dialkylamino, each of which has 1 to 6 carbon atoms in the alkyl groups and is optionally substituted by halogen, cyano or C₁-C₄-alkoxy, or represents cycloalkyl having 3 to 6 carbon atoms which is optionally substituted by halogen, cyano, carboxyl, C₁-C₄-alkyl or C₁-C₄-alkoxycarbonyl, and
R⁵ represents hydrogen, hydroxyl, cyano, or represents alkyl having 1 to 6 carbon atoms which is optionally substituted by halogen, cyano or C₁-C₄-alkoxy, or represents cycloalkyl having 3 to 6 carbon atoms which is optionally substituted by halogen, cyano, carboxyl, C₁-C₄-alkyl or C₁-C₄-alkoxycarbonyl,
Z represents alkyl having 1 to 8 carbon atoms which is optionally substituted by halogen, cyano, hydroxyl, amino, C₁-C₄-alkoxy, C₁-C₄-alkylthio, C₁-C₄-alkylsulphinyl or C₁-C₄-alkylsulphonyl (which are in each case optionally substituted by halogen), or represents alkenyl or alkinyl, each of which has up to 8 carbon atoms and is optionally substituted by halogen, or represents cycloalkyl having 3 to 6 carbon atoms which is optionally substituted by halogen, cyano, carboxyl, phenyl (which is optionally substituted by halogen, cyano, C₁-C₄-alkyl, C₁-C₄-halogenoalkyl, C₁-C₄-alkoxy or C₁-C₄-halogenoalkoxy), C₁-C₄-alkyl or C₁-C₄-alkoxycarbonyl, or represents in each case optionally substituted phenyl, naphthyl or (optionally benzo-fused) heterocyclyl having 5 or 6 ring members of which at least one represents oxygen, sulphur or nitrogen and optionally one or two further ring members represent nitrogen, the substituents which are possible being selected from the enumeration which follows:
oxygen (as a replacement for two geminal hydrogen atoms), halogen, cyano, nitro, amino, hydroxyl, formyl, carboxyl, carbamoyl, thiocarbamoyl, in each case straight-chain or branched alkyl, alkoxy, alkylthio, alkylsulphinyl or alkylsulphonyl, each of which has 1 to 6 carbon atoms, in each case straight-chain or branched alkenyl or alkenyloxy, each of which has 2 to 6 carbon atoms, in each case straight-chain or branched halogenoalkyl, halogenoalkoxy, halogenoalkylthio, halogenoalkylsulphinyl or halogenoalkylsulphonyl, each of which has 1 to 6 carbon atoms and 1 to 13 identical or different halogen atoms, in each case straight-chain or branched halogenoalkenyl or halogenoalkenyloxy, each of which has 2 to 6 carbon atoms and 1 to 13 identical or different halogen atoms, in each case straight-chain or branched alkylamino, dialkylamino, alkylcarbonyl, alkylcarbonyloxy, alkoxycarbonyl, alkylsulphonyloxy, hydroximinoalkyl or alkoximinoalkyl, each of which has 1 to 6 carbon atoms in the individual alkyl moieties, in each case divalent alkylene or dioxyalkylene, each of which has 1 to 6 carbon atoms and each of which is optionally monosubstituted or polysubstituted by identical or different substituents from the series consisting of halogen and/or straight-chain or branched alkyl having 1 to 4 carbon atoms and/or straight-chain or branched halogenoalkyl having 1 to 4 carbon atoms and 1 to 9 identical or different halogen atoms, or cycloalkyl having 3 to 6 carbon atoms, heterocyclyl or heterocyclyl-methyl, each of which has 3 to 7 ring members of which in each case 1 to 3 are identical or different hetero atoms - in particular nitrogen, oxygen and/or sulphur -, or phenyl, phenoxy, benzyl, benzyloxy, phenylethyl or phenylethyloxy, each of which is optionally monosubstituted or polysubstituted in the phenyl moiety by identical or different substituents from the series consisting of halogen, cyano, nitro, carboxyl, carbamoyl and/or straight-chain or branched alkyl having 1 to 4 carbon atoms and/or straight-chain or branched halogenoalkyl having 1 to 4 carbon atoms and 1 to 9 identical or different halogen atoms and/or straight-chain or branched alkoxy having 1 to 4 carbon atoms and/or straight-chain or branched halogenoalkoxy having 1 to 4 carbon atoms and 1 to 9 identical or different halogen atoms and/or alkylcarbonyl or alkoxycarbonyl, each of which has up to 5 carbon atoms.

3. Compounds of the formula (I) according to Claim 1, in which
R represents cyano;
X represents methoxy, ethoxy, n- or i-propoxy, n-, i-, s- or t-butoxy; methylthio, ethylthio, n- or i-propylthio, n-, i-, s- or t-butylthio; amino, methylamino, ethylamino, n- or i-propylamino; dimethylamino, methyl-ethylamino, diethylamino, methyl-n- or i-propylamino and ethyl-n- or i-propylamino;
X and R together with the carbon or nitrogen atom to which they are bonded represent a triazole radical of the formula 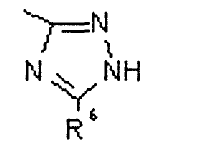 where
R⁶ represents methyl, ethyl, n- or i-propyl, n-, i-, s- or t-butyl; hydroxyl; mercapto; amino, methylamino, ethylamino, n- or i-propylamino; dimethylamino, methyl-ethyl-amino, diethylamino, methyl-n- or i-propylamino and ethyl-n- or i-propylamino;
Ar represents in each case optionally substituted ortho-, meta- or para-phenylene, or represents furanediyl, thiophenediyl, pyrrolediyl, pyrazolediyl, triazolediyl, oxazolediyl, isoxazolediyl, thiazolediyl, isothiazolediyl, oxadiazolediyl, thiadiazolediyl, pyridinediyl, pyrimidinediyl, pyridazinediyl, pyrazinediyl, 1,3,4-triazinediyl or 1,2,3-triazinediyl, the substituents which are possible being selected in particular amongst those of the enumeration which follows:
fluorine, chlorine, cyano, methyl, ethyl, trifluoromethyl, methoxy, ethoxy, methylthio, methylsulphinyl or methylsulphonyl;
G represents a single bond, represents oxygen or represents in each case optionally fluorine-, chlorine-, hydroxyl-, methyl-, ethyl-, n- or i-propyl-, trifluoromethyl-, cyclopropyl-, cyclobutyl-, cyclopentyl- or cyclohexyl-substituted methylene, dimethylene (ethane-1,2-diyl), ethene-1,2-diyl, ethine-1,2-diyl or one of the groups which follow
-Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q- or -N(R⁵)-CQ-Q-CH₂-, where
n represents the numbers 0, 1 or 2,
Q represents oxygen or sulphur,
R⁴ represents hydrogen, cyano, or represents methyl, ethyl, n- or i-propyl, n-, i- or s-butyl, methoxy, ethoxy, propoxy, butoxy, methylthio, ethylthio, propylthio, butylthio, methylamino, ethylamino, propylamino, dimethylamino or diethylamino, which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, n- or i-propyl, methoxycarbonyl or ethoxycarbonyl, and
R⁵ represents hydrogen, hydroxyl, cyano, or represents methyl, ethyl, n- or i-propyl or n-, i-, s- or t-butyl, each of which is optionally substituted by fluorine, chlorine, cyano, methoxy or ethoxy, or represents cyclopropyl, cyclobutyl, cyclopentyl or cyclohexyl, each of which is optionally substituted by fluorine, chlorine, cyano, carboxyl, methyl, ethyl, n- or i-propyl, methoxycarbonyl or ethoxycarbonyl;
Z represents methyl, ethyl, n- or i-propyl or n-, i-, s- or t-butyl, which is optionally substituted by fluorine, chlorine, bromine, cyano, hydroxyl, amino, methoxy, ethoxy, methylthio, ethylthio, methylsulphinyl, ethylsulphinyl, methylsulphonyl or ethylsulphonyl (which are in each case optionally substituted by fluorine and/or chlorine), or represents allyl, crotonyl, 1-methyl-allyl, propargyl or 1-methyl-propargyl, each of which is optionally substituted by fluorine, chlorine or bromine, or represents cyclopropyl, cyclobutyl, cyclopentyl or cyclohexyl, each of which is optionally substituted by fluorine, chlorine, bromine, cyano, carboxyl, phenyl (which is optionally substituted by fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy), methyl, ethyl, n- or i-propyl, methoxycarbonyl or ethoxycarbonyl, or represents in each case optionally substituted phenyl, naphthyl, furyl, tetrahydrofuryl, benzofuryl, tetrahydropyranyl, thienyl, benzothienyl, pyrrolyl, dihydropyrrolyl, tetrahydropyrrolyl, benzopyrrolyl, benzodihydropyrrolyl, oxazolyl, benzoxazolyl, isoxazolyl, thiazolyl, benzothiazolyl, isothiazolyl, imidazolyl, benzimidazolyl, oxadiazolyl, thiadiazolyl, pyridinyl, pyrimidinyl, pyridazinyl, pyrazinyl, 1,2,3-triazinyl, 1,2,4-triazinyl or 1,3,5-triazinyl, the substituents which are possible being selected from the enumeration which follows:
oxygen (as a replacement for two geminal hydrogen atoms), fluorine, chlorine, bromine, cyano, nitro, amino, hydroxyl, formyl, carboxyl, carbamoyl, thiocarbamoyl, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methylamino, ethylamino, n- or i-propylamino, dimethylamino, diethylamino, acetyl, propionyl, acetyloxy, methoxycarbonyl, ethoxycarbonyl, methylsulphonyloxy, ethylsulphonyloxy, hydroximinomethyl, hydroximinoethyl, methoximinomethyl, ethoximinomethyl, methoximinoethyl or ethoximinoethyl; trimethylene (propane-1,3-diyl), methylenedioxy or ethylenedioxy, cyclopropyl, cyclobutyl, cyclopentyl or cyclohexyl, each of which is optionally monosubstituted or polysubstituted by identical or different substituents from the series consisting of fluorine, chlorine, methyl, ethyl or n- or i-propyl, and phenyl, phenoxy, benzyl or benzyloxy, each of which is optionally monosubstituted or polysubstituted in the phenyl moiety by identical or different substituents from the series consisting of fluorine, chlorine, bromine, cyano, nitro, carboxyl, carbamoyl, methyl, ethyl, n- or i-propyl, n-, i-, s- or t-butyl, trifluoromethyl, methoxy, ethoxy, n- or i-propoxy, difluoromethoxy, trifluoromethoxy, acetyl, methoxycarbonyl or ethoxycarbonyl.

4. Compounds of the formula (I) according to Claim 1, in which
R represents cyano;
m represents the numbers 0, 1 or 2;
X represents methoxy, ethoxy, methylthio, ethylthio, amino, methylamino, ethylamino, dimethylamino or diethylamino;
X and R together with the carbon or nitrogen atom to which they are bonded represent a triazole radical of the formula 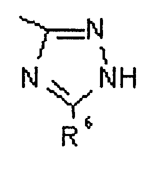 where
R⁶ represents methyl, ethyl, hydroxyl, mercapto or amino;
Ar represents ortho-phenylene, pyridine-2,3-diyl or thiophene-2,3-diyl;
G represents oxygen, methylene or one of the groups which follow
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -O-C(R⁴)=N-O-CH₂-, -NH-C(R⁴)=N-O-CH₂-, -N(R⁵)- or -CH₂-O-N=C(R⁴)-,
where
n represents the numbers 0, 1 or 2,
R⁴ represents hydrogen, methyl or ethyl and
R⁵ represents hydrogen, methyl or ethyl;
Z represents in each case optionally substituted phenyl, pyridinyl, pyrimidinyl, pyridazinyl, pyrazinyl, 1,2,3-triazinyl, 1,2,4-triazinyl or 1,3,5-triazinyl, the substituents which are possible being selected from the enumeration which follows:
fluorine, chlorine, bromine, cyano, methyl, ethyl, n- or i-propyl, n-, i-, s- or t-butyl, methoxy, ethoxy, n- or i-propoxy, methylthio, ethylthio, n- or i-propylthio, methylsulphinyl, ethylsulphinyl, methylsulphonyl or ethylsulphonyl, trifluoromethyl, difluoromethoxy, trifluoromethoxy, difluoromethylthio, trifluoromethylthio, trifluoromethylsulphinyl or trifluoromethylsulphonyl, methoxycarbonyl, ethoxycarbonyl, methoximinomethyl, ethoximinomethyl, methoximinoethyl, ethoximinoethyl, methylenedioxy or ethylenedioxy, each of which is optionally monosubstituted or polysubstituted by identical or different substituents from the series consisting of fluorine, chlorine, methyl or ethyl, or phenyl, phenoxy, benzyl or benzyloxy, each of which is optionally monosubstituted or polysubstituted in the phenyl moiety by identical or different substituents from the series consisting of fluorine, chlorine, bromine, cyano, methyl, ethyl, n- or i-propyl, n-, i-, s- or t-butyl, trifluoromethyl, methoxy, ethoxy, n- or i-propoxy, difluoromethoxy or trifluoromethoxy.

5. Compoounds of the formula (I) according to Claim 1 in which
Ar represents ortho-phenylene.

6. Pesticides, characterized in that they comprise at least one compound of the formula (I) according to Claim 1.

7. Methods of combating pests, characterized in that compounds of the formula (I) according to Claim 1 are allowed to act on pests and/or their environment.

8. Process for the preparation of compounds of the formula (I) according to Claim 1, characterized in that
a) nitriles of the general formula (II) 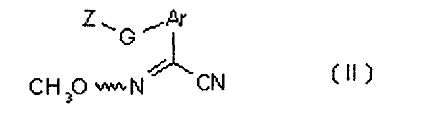 in which
Ar, G and Z have the meanings given in Claim 1
are reacted with (thio)alcohols of the general formula (III)
H-X¹ (III)
in which
X¹ represents alkoxy or alkylthio, or with their alkali metal salts, if appropriate in the presence of a diluent and if appropriate in the presence of a reaction auxiliary;
or
b) the imidates which can be obtained by process (a) which have the general formula (Ia) 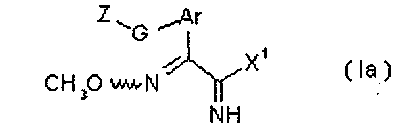 in which
Ar, G, Z and X¹ have the abovementioned meanings,
are reacted with reactive acid derivatives, in particular with acid halides of the general formula (IV)
Hal-R (IV)
in which
R has the meaning given in Claim 1 and
Hal represents halogen,
or with the corresponding anhydrides, if appropriate in the presence of a diluent and if appropriate in the presence of a reaction auxiliary;
or
c) the imidates which can be obtained by process (a) which have the general formula (Ia) 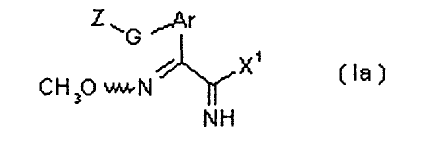 in which
Ar, G, Z and X¹ have the abovementioned meanings,
are reacted with cyanamide, of the formula (V),
NH₂-CN (V)
if appropriate in the presence of a diluent and if appropriate in the presence of a reaction auxiliary;
or
d) the imidic acid derivatives which can be obtained by processes (b) and (c) which have the general formula (Ib) 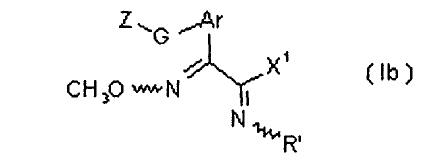 in which
Ar, G, Z and X¹ have the abovementioned meanings and
R' represents cyano,
are reacted with amines of the general formula (VI)
H-X² (VI)
in which
X² represents amino, alkylamino or dialkylamino,
if appropriate in the presence of a diluent;
or
e) the imidic acid derivatives which can be obtained by processes (b) and (c) which have the general formula (Ic) 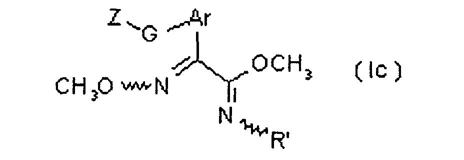 in which
Ar, G, Z and R' have the abovementioned meanings,
are reacted with hydrazine or hydrazine hydrate, if appropriate in the presence of a diluent;
or
f) the imidates which can be obtained by process (a) which have the general formula (Ia) 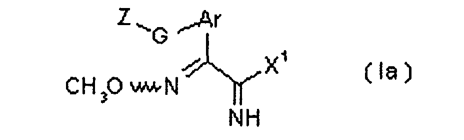 in which Ar, G, Z and X¹ have the abovementioned meanings,
are reacted with amines of the general formula (VI)
H-X² (VI)
in which
X² has the abovementioned meaning,
if appropriate in the presence of a diluent.

9. Use of compounds of the formula (I) according to Claims 1 to 5 for combating pests.

10. Process for the preparation of pesticides, characterized in that compounds of the formula (I) according to Claims 1 to 5 are mixed with extenders and/or surface-active agents.

11. Compounds of the formula (Ia) 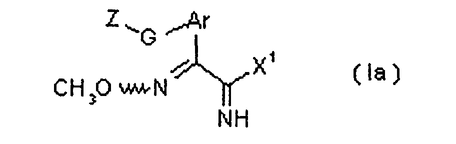 in which
Ar, G and Z have the meanings given in Claim 1
and
X¹ represents alkoxy or alkylthio.

12. Compounds of the formula (Ib) 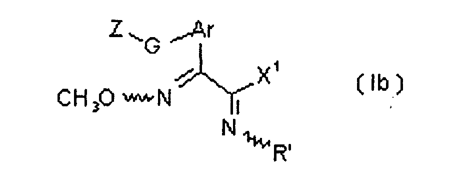 in which
Ar, G, Z and X¹ have the meanings given in Claim 11 and
R' represents cyano.

13. Compounds of the formula (Ic) 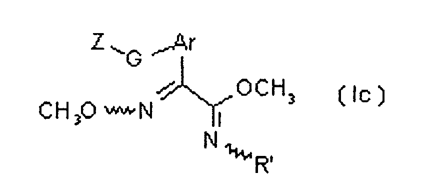 in which
Ar, G, Z and R' have the meanings given in Claim 12.

## Revendications

1. Dérivés d'imido-acides de formule générale (I), 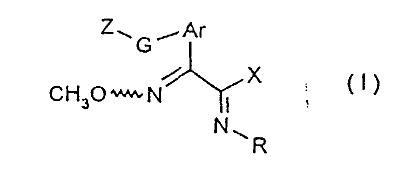 dans laquelle
R est un groupe cyano ;
X est un groupe alkoxy, alkylthio, amino, alkylamino ou dialkylamino,
ou bien
X et R forment conjointement avec l'atome de carbone ou l'atome d'azote auquel ils sont liés un reste triazole de formule 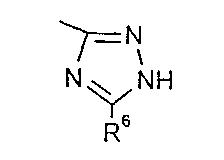 dans laquelle
R⁶ est un groupe alkyle, hydroxy, mercapto, amino, alkylamino ou dialkylamino ;
Ar est un groupe arylène ou un groupe hétéroarylène dont chacun est éventuellement substitué ;
G représente une liaison simple, l'oxygène, un groupe alcanediyle, alcènediyle, oxa-alcènediyle, alcynediyle dont chacun est éventuellement substitué par un halogène, un radical hydroxy, alkyle, halogénalkyle ou cycloalkyle, ou représente l'un des groupements suivants :
Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂, -NH-C(R⁴)=N-O-CH₂-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q, -CQ-N-(R⁵)-CQ-Q-, -N(R⁵)-CQ-Q-CH₂-,-CQ-CH₂- ou -N=N-C(R⁴)=N-O-,
dans lesquels
Q représente l'oxygène ou le soufre,
n représente les nombres 0, 1 ou 2,
R⁴ est l'hydrogène, un groupe cyano ou un groupe alkyle, alkoxy, alkylthio, alkylamino, dialkylamino ou cycloalkyle éventuellement substitué dans chaque cas et
R⁵ représente l'hydrogène, un groupe hydroxy, cyano ou un groupe alkyle, alkoxy ou cycloalkyle éventuellement substitué dans chaque cas ; et
Z représente un groupe alkyle, alcényle, alcynyle, cycloalkyle, aryle ou hétérocycloalkyle éventuellement substitué dans chaque cas ;
en excluant les composés dans lesquels, en même temps, R représente l'hydrogène, G est l'oxygène ou le groupement -OCH₂- et Z représente un groupe aryle ou un groupe 2-pyridyle dont chacun est éventuellement substitué.

2. Composés de formule (I) suivant la revendication 1, dans laquelle
R est un groupe cyano ;
X représente un groupe alkoxy ou un groupe alkylthio ayant chacun 1 à 4 atomes de carbone et étant chacun linéaire ou ramifié ;
un groupe amino ou bien des groupes alkylamino et dialkylamino ayant chacun 1 à 4 atomes de carbone dans les parties alkyle individuelles et étant chacun linéaire ou ramifié,
X et R forment conjointement avec l'atome de carbone ou d'azote auquel ils sont liés un reste triazole de formule 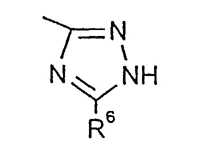 dans laquelle
R⁶ est un groupe alkyle linéaire ou ramifié ayant 1 à 4 atomes de carbone, un groupe hydroxy, mercapto, amino, ou bien des groupes alkylamino et dialkylamino ayant chacun 1 à 4 atomes de carbone dans les parties alkyle individuelles et étant chacun liénaire ou ramifié,
Ar représente un groupe phénylène ou naphtylène dont chacun est éventuellement substitué ou un groupe hétéroarylène à noyau de 5 ou 6 atomes, dont l'un au moins est un atome d'oxygène, de soufre ou d'azote et, le cas échéant, un ou deux autres représentent de l'azote, les substituants possibles étant choisis parmi les suivants : halogène, cyano, nitro, amino, hydroxy, formyle, carboxy, carbamoyle, thiocarbamoyle ; alkyle, alkoxy, alkylthio, alkylsulfinyle ou alkylsulfonyle ayant chacun 1 à 6 atomes de carbone et étant chacun linéaire ou ramifié ; alcényle, alcényloxy ou alcynyloxy ayant chacun 2 à 6 atomes de carbone et étant chacun linéaire ou ramifié ; halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ; halogénalcényle ou halogénalcényloxy étant chacun linéaire ou ramifié et ayant chacun 2 à 6 atomes de carbone et 1 à 13 atomes d'halogènes identiques ou différents ; alkylamino, dialkylamino, alkylcarbonyle, alkylcarbonyloxy, alkoxycarbonyle, alkylsulfonyloxy, hydroximinoalkyle ou alkoximinoalkyle ayant chacun 1 à 6 atomes de carbone dans les parties alkyle individuelles et étant chacun linéaire ou ramifié ; alkylène ou dioxyalkylène ayant chacun 1 à 6 atomes de carbone et ayant chacun deux liaisons, portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents,
G représente une liaison simple, l'oxygène, un groupe alcanediyle, alcènediyle, oxa-alcènediyle, alcynediyle ayant chacun 4 atomes de carbone et portant chacun, le cas échéant, un substituant halogéno, hydroxy, alkyle en C₁ à C₄, halogénalkyle en C₁ à C₄ ou cycloalkyle en C₃ à C₆, ou bien l'un des groupements suivants :
Q-CQ-, -CQ-Q-, -CH₂-Q-; -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-, -N(R⁵)-CQ-Q-, -CQ-N(R⁵)-CQ-Q-, -N(R⁵)-CQ-Q-CH₂-, -CQ-CH₂- ou -N=N-C(R⁴)=N-,
dans lesquels
n représente les nombres 0, 1 ou 2,
Q est l'oxygène ou le soufre,
R⁴ est l'hydrogène, un groupe cyano, un groupe alkyle, alkoxy, alkylthio, alkylamino ou dialkylamino ayant chacun 1 à 6 atomes de carbone dans les groupes alkyle et portant chacun, le cas échéant, un substituant halogéno, cyano ou alkoxy en C₁ à C₄, ou bien un groupe cycloalkyle ayant 3 à 6 atomes de carbone éventuellement substitué par un radical halogéno, cyano, carboxy, alkyle en C₁ à C₄ ou (alkoxy en C₁ à C₄)-carbonyle, et
R⁵ représente l'hydrogène, un groupe hydroxy, cyano ou un groupe alkyle ayant 1 à 6 atomes de carbone éventuellement substitué par un radical halogéno, cyano ou alkoxy en C₁ à C₄, ou un groupe cycloalkyle de 3 à 6 atomes de carbone éventuellement substitué par un radical halogéno, cyano, carboxy, alkyle en C₁ à C₄ ou alkoxy en C₁ à C₄,
Z représente un groupe alkyle ayant 1 à 8 atomes de carbone éventuellement substitué par un radical halogéno, cyano, hydroxy, amino, alkoxy en C₁ à C₄, alkylthio en C₁ à C₄, alkylsulfinyle en C₁ à C₄ ou alkylsulfonyle en C₁ à C₄ (chacun pouvant être substitué par un halogène) ; un groupe alcényle ou alcynyle ayant chacun jusqu'à 8 atomes de carbone et portant chacun, le cas échéant, un substituant halogéno ; un groupe cycloalkyle de 3 à 6 atomes de carbone éventuellement substitué par un radical halogéno, cyano, carboxy, phényle (qui est substitué, le cas échéant, par un radical halogéno, cyano, alkyle en C₁ à C₄, halogénalkyle en C₁ à C₄, alkoxy en C₁ à C₄ ou halogénalkoxy en C₁ à C₄), alkyle en C₁ à C₄ ou (alkoxy en C₁ à C₄)-carbonyle ; un groupe, éventuellement substitué dans chaque cas, phényle, naphtyle ou hétérocyclyle (éventuellement condensé au benzène) à noyau de 5 ou 6 atomes, dont l'un au moins est un atome d'oxygène, de soufre ou d'azote et, le cas échéant, un ou deux autres représentent de l'azote, les substituants possibles étant choisis parmi les suivants :
oxygène (en remplacement de deux atomes géminés d'hydrogène), halogène, cyano, nitro, amino, hydroxy, formyle, carboxy, carbamoyle, thiocarbamoyle ; alkyle, alkoxy, alkylthio, alkylsulfinyle ou alkylsulfonyle étant chacun linéaire ou ramifié et ayant chacun 1 à 6 atomes de carbone ; alcényle ou alcényloxy étant chacun linéaire ou ramifié et ayant chacun 2 à 6 atomes de carbone ; halogénalkyle, halogénalkoxy, halogénalkylthio, halogénalkylsulfinyle ou halogénalkylsulfonyle étant chacun linéaire ou ramifié et ayant chacun 1 à 6 atomes de carbone et 1 à 13 atomes d'halogènes identiques ou différents ; halogénalcényle ou halogénalcényloxy étant chacun linéaire ou ramifié et ayant chacun 2 à 6 atomes de carbone et 1 à 13 atomes d'halogènes identiques ou différents ; alkylamino, dialkylamino, alkylcarbonyle, alkylcarbonyloxy, alkoxycarbonyle, alkylsulfonyloxy, hydroximinoalkyle ou alkoximinoalkyle étant chacun linéaire ou ramifié et ayant chacun 1 à 6 atomes de carbone dans les parties alkyle individuelles ; alkylène ou dioxyalkylène ayant chacun 1 à 6 atomes de carbone et chacun deux liaisons, portant chacun, le cas échéant, un ou plusieurs substituants, identiques ou différents, halogéno et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents ; cycloalkyle ayant 3 à 6 atomes de carbone ; hétérocyclyle ou hétérocyclylméthyle ayant chacun un noyau de 3 à 7 atomes dont chaque fois un à trois sont des hétéroatomes identiques ou différents - notamment azote, oxygène et/ou soufre -, ainsi que phényle, phénoxy, benzyle, benzyloxy, phényléthyle ou phényléthyloxy portant chacun, le cas échéant dans la partie phényle un ou plusieurs substituants, identiques ou différents, halogéno, cyano, nitro, carboxy, carbamoyle et/ou alkyle linéaire ou ramifié ayant 1 à 4 atomes de carbone et/ou halogénalkyle linéaire ou ramifié ayant 1 à 4 atomes de carbone et 1 à 9 atomes d'halogènes identiques ou différents et/ou alkoxy linéaire ou ramifié ayant 1 à 4 atomes de carbone et/ou halogénalkoxy linéaire ou ramifié ayant 1 à 4 atomes de carbone et 1 à 9 atomes d'halogènes identiques ou différents et/ou alkylcarbonyle ou alkoxycarbonyle ayant chacun jusqu'à 5 atomes de carbone.

3. Composés de formule (I) suivant la revendication 1, dans laquelle
R est un groupe cyano ;
X est un groupe méthoxy, éthoxy, n-propoxy, isopropoxy, n-butoxy, isobutoxy, sec.-butoxy, tertio-butoxy, méthylthio, éthylthio, n-propylthio, isopropylthio, n-butylthio, isobutylthio, sec.-butylthio, tertio-butylthio, amino, méthylamino, éthylamino, n-propylamino, isopropylamino, diméthylamino, méthyléthylamino, diéthylamino, méthyl-n-propylamino, méthylisopropylamino, éthyl-n-propylamino ou éthylisopropylamino,
X et R forment conjointement avec l'atome de carbone ou d'azote auquel ils sont liés un reste triazole de formule 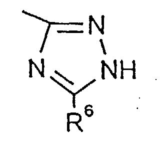 dans laquelle
R⁶ est un groupe méthyle, éthyle, n-propyle, isopropyle, n-butyle, isobutyle, sec.-butyle, tertio-butyle, hydroxy, mercapto, amino, méthylamino, éthylamino, n-propylamino, isopropylamino, diméthylamino, méthyléthylamino, diéthylamino, méthyl-n-propylamino, isopropylamino, éthyl-n-propylamino ou éthylisopropylamino,
Ar représente un groupe ortho-, méta- ou para-phénylène éventuellement substitué dans chaque cas, un groupe furannediyle, thiophènediyle, pyrrolediyle, pyrazolediyle, triazolediyle, oxazolediyle, isoxazolediyle, thiazolediyle, isothiazolediyle, oxadiazolediyle, thiadiazolediyle, pyridinediyle, pyrimidinediyle, pyridazinediyle, pyrazinediyle, 1,3,4-triazinediyle ou 1,2,3-triazinediyle, les substituants possibles étant choisis en particulier parmi les suivants :
fluor, chlore, cyano, méthyle, éthyle, trifluorométhyle, méthoxy, éthoxy, méthylthio, méthylsulfinyle ou méthylsulfonyle,
G représente une liaison simple, l'oxygène, un groupe méthylène, diméthylène (éthane-1,2-diyle), éthène-1,2-diyle, éthyne-1,2-diyle portant chacun, le cas échéant, un substituant fluoro, chloro, hydroxy, méthyle, éthyle, n-propyle, isopropyle, trifluorométhyle, cyclopropyle, cyclobutyle, cyclopentyle ou cyclohexyle, ou bien l'un des groupements suivants :
-Q-CQ-, -CQ-Q-, -CH₂-Q-, -Q-CH₂-, -CQ-Q-CH₂-, -CH₂-Q-CQ-, -Q-CQ-CH₂-, -Q-CQ-Q-CH₂-, -N=N-, -S(O)ₙ-, -CH₂-S(O)ₙ-, -CQ-, -S(O)ₙ-CH₂-, -C(R⁴)=N-O-, -C(R⁴)=N-O-CH₂-, -Q-C(R⁴)=N-O-CH₂-, -NH-C(R⁴)=N-O-CH²-, -N(R⁵)-, -CQ-N(R⁵)-, -N(R⁵)-CQ-, -Q-CQ-N(R⁵)-, -N=C(R⁴)-Q-CH₂-, -CH₂-O-N=C(R⁴)-,-N(R⁵)-CQ-Q-, -CQ-N(R⁵)-CQ-Q- ou N(R⁵)-CQ-Q-CH₂-
dans lesquels
n représente les nombres 0, 1 ou 2,
Q est l'oxygène ou le soufre,
R⁴ représente l'hydrogène, un groupe cyano, un groupe méthyle, éthyle, n-propyle, isopropyle, n-butyle, isobutyle, sec.-butyle, méthoxy, éthoxy, propoxy, butoxy, méthylthio, éthylthio, propylthio, butylthio, méthylamino, éthylamino, propylamino, diméthylamino ou diéthylamino dont chacun est éventuellement substitué par du fluor, du chlore, un radical cyano, méthoxy ou éthoxy, ou bien un groupe cyclopropyle, cyclobutyle, cyclopentyle ou cyclohexyle dont chacun est substitué, le cas échéant, par du fluor, du chlore, un radical cyano, carboxy, méthyle, éthyle, n-propyle, isopropyle, méthoxycarbonyle ou éthoxycarbonyle, et
R⁵ représente l'hydrogène, un groupe hydroxy, cyano, ou un groupe méthyle, éthyle, n-propyle, isopropyle, n-butyle, isobutyle, sec.-butyle ou tertio-butyle dont chacun est substitué, le cas échéant, par du fluor, du chlore, un radical cyano, méthoxy ou éthoxy, ou bien un groupe cyclopropyle, cyclobutyle, cyclopentyle ou cyclohexyle substitué, le cas échéant, par du fluor, du chlore, un radical cyano, carboxy, méthyle, éthyle, n-propyle, isopropyle, méthoxycarbonyle ou éthoxycarbonyle,
Z représente un groupe méthyle, éthyle, n-propyle, isopropyle, n-butyle, isobutyle, sec.-butyle, tertio-butyle substitué, le cas échéant, par du fluor, du chlore, du brome, un radical cyano, hydroxy, amino, méthoxy, éthoxy, méthylthio, éthylthio, méthylsulfinyle, éthylsulfinyle, méthylsulfonyle, éthylsulfonyle (substitués chacun, le cas échéant, par du fluor et/ou du chlore) ; un groupe allyle, crotonyle, 1-méthylallyle, propargyle ou 1-méthylpropargyle dont chacun est substitué, le cas échéant, par du fluor, du chlore ou du brome ; un groupe cyclopropyle, cyclobutyle, cyclopentyle ou cyclohexyle dont chacun est substitué, le cas échéant, par du fluor, du chlore, du brome, un radical cyano, carboxy, phényle (qui est substitué, le cas échéant, par du fluor, du chlore, du brome, un radical cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy), méthyle, éthyle, n-propyle, isopropyle, méthoxycarbonyle ou éthoxycarbonyle ; un groupe phényle, naphtyle, furyle, tétrahydrofuryle, benzofuryle, tétrahydropyrannyle, thiényle, benzothiényle, pyrrolyle, dihydropyrrolyle, tétrahydropyrrolyle, benzopyrrolyle, benzodihydropyrrolyle, oxazolyle, benzoxazolyle, isoxazolyle, thiazolyle, benzthiazolyle, isothiazolyle, imidazolyle, benzimidazolyle, oxadiazolyle, thiadiazolyle, pyridinyle, pyrimidinyle, pyridazinyle, pyrazinyle, 1,2,3-triazinyle, 1,2,4-triazinyle ou 1,3,5-triazinyle, dont chacun est éventuellement substitué, les substituants possibles étant choisis parmi les suivants :
oxygène (en remplacement de deux atomes d'hydrogène géminés), fluor, chlore, brome, cyano, nitro, amino, hydroxy, formyle, carboxy, carbamoyle, thiocarbamoyle, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthylamino, éthylamino, n-propylamino, isopropylamino, diméthylamino, diéthylamino, acétyle, propionyle, acétyloxy, méthoxycarbonyle, éthoxycarbonyle, méthylsulfonyloxy, éthylsulfonyloxy, hydroximinométhyle, hydroximinoéthyle, méthoximinométhyle, éthoximinométhyle, méthoximinoéthyle ou éthoximinoéthyle ; triméthylène (propane-1,3-diyle), méthylènedioxy, éthylènedioxy, cyclopropyle, cyclobutyle, cyclopentyle ou cyclohexyle dont chacun est substitué, le cas échéant, une ou plusieurs fois identiques ou différentes par du fluor, du chlore, un radical méthyle, éthyle, n-propyle ou isopropyle, ainsi que phényle, phénoxy, benzyle ou benzyloxy dont chacun est substitué, le cas échéant, dans la partie phényle une ou plusieurs fois identiques ou différentes par du fluor, du chlore, du brome, un radical cyano, nitro, carboxy, carbamoyle, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy, trifluorométhoxy, acétyle, méthoxycarbonyle ou éthoxycarbonyle.

4. Composés de formule (I) suivant la revendication 1, dans laquelle
X est un groupe méthoxy, éthoxy, méthylthio, éthylthio, amino, méthylamino, éthylamino, diméthylamino ou diéthylamino,
X et R forment conjointement avec l'atome de carbone ou d'azote auquel ils sont liés un reste triazole de formule 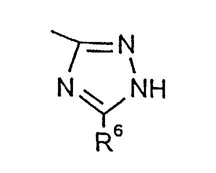 dans laquelle
R⁶ est un groupe méthyle, éthyle, hydroxy, mercapto ou amino,
Ar est un groupe ortho-phénylène, pyridine-2,3-diyle ou thiophène-2,3-diyle,
G représente l'oxygène, un groupe méthylène ou l'un des groupements suivants :
-CH₂-O-, -O-CH₂-, -S(O)ₙ-, -CH₂-S(O)ₙ-, -S(O)ₙ-CH₂-, -C(R⁴)=N-O-, -O-N=C(R⁴)-, -C(R⁴)=N-O-CH₂-, -O-C(R⁴)=N-O-CH₂-, -NH-C(R⁴)=N-O-CH₂-, -N(R⁵)- ou -CH₂-O-N=C(R⁴)-
dans lesquels
n représente les nombres 0, 1 ou 2,
R⁴ est l'hydrogène, un groupe méthyle ou éthyle et
R⁵ est l'hydrogène, un groupe méthyle ou éthyle,
Z représente un groupe phényle, pyridinyle, pyrimidinyle, pyridazinyle, pyrazinyle, 1,2,3-triazinyle, 1,2,4-triazinyle ou 1,3,5-triazinyle dont chacun est éventuellement substitué, les substituants possibles étant choisis parmi les suivants :
fluor, chlore, brome, cyano, méthyle, éthyle, n-propyle, isopropyle, n-butyle, isobutyle, sec.-butyle, tertio-butyle, méthoxy, éthoxy, n-propoxy, isopropoxy, méthylthio, éthylthio, n-propylthio, isopropylthio, méthylsulfinyle, éthylsulfinyle, méthylsulfonyle, éthylsulfonyle, trifluorométhyle, difluorométhoxy, trifluorométhoxy, difluorométhylthio, trifluorométhylthio, trifluorométhylsulfinyle, trifluorométhylsulfonyle, méthoxycarbonyle, éthoxycarbonyle, méthoximinométhyle, éthoximinométhyle, méthoximinoéthyle, éthoximinoéthyle, méthylènedioxy ou éthylènedioxy portant chacun, le cas échéant, un ou plusieurs substituants, identiques ou différents, fluor, chlore, méthyle ou éthyle, ainsi que phényle, phénoxy, benzyle ou benzyloxy portant chacun, le cas échéant, dans la partie phényle un ou plusieurs substituants, identiques ou différents, fluor, chlore, brome, cyano, méthyle, éthyle, n-propyle, isopropyle, n-butyle, isobutyle, sec.-butyle, tertio-butyle, trifluorométhyle, méthoxy, éthoxy, n-propoxy, isopropoxy, difluorométhoxy ou trifluorométhoxy.

5. Composés de formule (I) suivant la revendication 1, dans laquelle
Ar est un groupe ortho-phénylène.

6. Compositions pesticides, caractérisées par une teneur en au moins un composé de formule (I) suivant la revendication 1.

7. Procédé pour combattre des parasites, caractérisé en ce qu'on fait agir des composés de formule (I) suivant la revendication 1 sur des parasites et/ou sur leur milieu.

8. Procédé de production de composés de formule (I) suivant la revendication 1, caractérisé en ce que
a) on fait réagir des nitriles de formule générale (II) 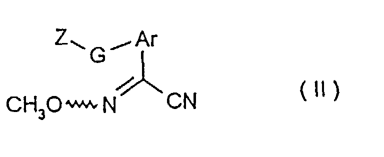 dans laquelle
Ar, G et Z ont la définition indiquée dans la revendication 1,
avec des (thio)alcools de formule générale (III)
H-X¹ (III)
dans laquelle
X¹ est un groupe alkoxy ou alkylthio,
ou leurs sels de métaux alcalins, le cas échéant en présence d'un diluant et en la présence éventuelle d'un auxiliaire de réaction ;
ou bien
b) on fait réagir les imido-esters obtenus selon le procédé (a), de formule générale (Ia) 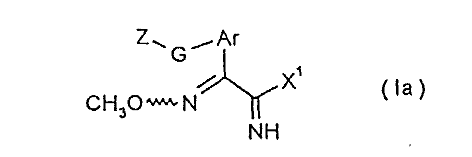 dans laquelle
Ar, G, Z et X¹ ont la définition indiquée ci-dessus,
avec des dérivés d'acides réactifs, notamment avec des halogénures d'acides de formule générale (IV)
Hal-R (IV)
dans laquelle
R a la définition indiquée dans la revendication 1 et
Hal représente un halogène,
ou avec les anhydrides correspondants, éventuellement en présence d'un diluant et, le cas échéant, en présence d'un auxiliaire de réaction,
ou bien
c) on fait réagir les imido-esters obtenus selon le procédé (a) de formule générale (Ia) 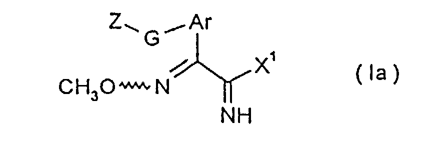 dans laquelle
Ar, G, Z et X¹ ont la définition indiquée ci-dessus,
avec le cyanamide de formule (V)
NH₂-CN (V)
éventuellement en présence d'un diluant et en la présence éventuelle d'un auxiliaire de réaction,
ou bien
d) on fait réagir les dérivés d'imido-acides obtenus selon les procédés (b) et (c), de formule générale (Ib) 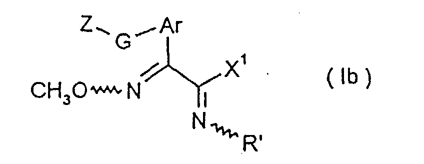 dans laquelle
Ar, G, Z et X¹ ont la définition indiquée ci-dessus, et
R' est un groupe cyano,
avec des amines de formule générale (VI)
H-X² (VI)
dans laquelle
X² est un groupe amino, alkylamino ou dialkylamino,
le cas échéant en présence d'un diluant ;
ou bien
e) on fait réagir les dérivés d'imido-acides obtenus selon les procédés (b) et (c), de formule générale (Ic) 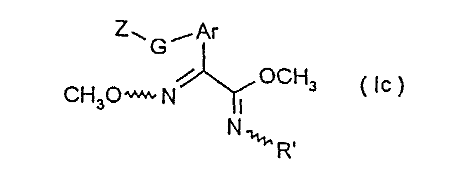 dans laquelle
Ar, G, Z et R' ont la définition indiquée ci-dessus,
avec l'hydrazine ou l'hydrate d'hydrazine, éventuellement en présence d'un diluant ;
ou bien
f) on fait réagir les imido-esters obtenus selon le procédé (a) de formule générale (la) 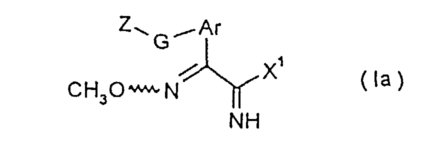 dans laquelle
Ar, G, Z et X¹ ont la définition indiquée ci-dessus,
avec des amines de formule générale (VI)
H-X² (VI)
dans laquelle
X² a la définition indiquée ci-dessus,
le cas échéant en présence d'un diluant.

9. Utilisation de composés de formule (I) suivant les revendications 1 à 5, pour combattre des parasites.

10. Procédé de préparation de compositions pesticides, caractérisé en ce qu'on mélange des composés de formule (I) suivant les revendications 1 à 5, avec des diluants et/ou des agents tensio-actifs.

11. Composés de formule (Ia) 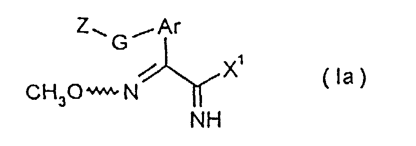 dans laquelle
Ar, G et Z ont la définition indiquée dans la revendication 1 et
X¹ est un groupe alkoxy ou alkylthio.

12. Composés de formule (Ib) 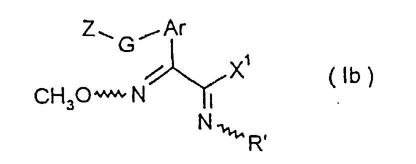 dans laquelle
Ar, G, Z et X¹ ont la définition indiquée dans la revendication 11 et
R' est un groupe cyano.

13. Composés de formule (Ic) 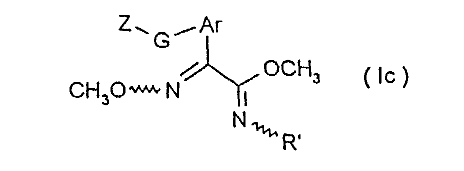 dans laqwuelle
Ar, G, Z et R' ont la définition indiquée dans la revendication 12.
